# EUROPEAN PATENT APPLICATION

(11) **EP 2 650 013 A1**
(43) Date of publication of application: **16.10.2013**
(21) Application number: 12163606.2
(22) Date of filing: 10.04.2012
(51) Int. Cl.: A61K 38/17, A61P 35/00

(54) **Inhibitors of Receptor for Advanced Glycation-End products (RAGE) for use in treating and/or preventing inflammation- and/or damage-induced cancer**

(71) Applicant: Deutsches Krebsforschungszentrum, 69120 Heidelberg (DE); Ruprecht-Karls-Universität Heidelberg, 69117 Heidelberg (DE); The Hebrew University of Jerusalem, 91905 Jerusalem (IL); HADASSAH MEDICAL ORGANIZATION, IL-91120 Jerusalem (IL)
(72) Inventor: The designation of the inventor has not yet been filed
(74) Representative: Dick, Alexander

(57) **Abstract**

The present invention is concerned with measures for tumor therapy. In particular, contemplated is an inhibitor of Receptor for Advanced Glycation-End products (RAGE) for use in treating and/or preventing inflammation- and/or damage-induced cancer. The invention, furthermore, relates to a medicament comprising said inhibitor of RAGE and, preferably, a pharmaceutically acceptable carrier. Further, the invention encompasses a method for identifying a subject in need of treatment with an inhibitor of RAGE, comprising determining in a sample from said subject the amount of RAGE, comparing said amount of RAGE determined in step a) with a reference amount, and identifying a subject in need of treatment with an inhibitor of RAGE. The invention also relates to a device for identifying a subject in need of treatment with an inhibitor of RAGE, as well as a kit adapted to carry out the method of the present invention, said kit comprising instructions for carrying out the aforementioned method.

## Description

The present invention is concerned with measures for tumor therapy. In particular, contemplated is an inhibitor of Receptor for Advanced Glycation-End products (RAGE) for use in treating and/or preventing inflammation- and/or damage-induced cancer. The invention, furthermore, relates to a medicament comprising said inhibitor of RAGE and, preferably, a pharmaceutically acceptable carrier. Further, the invention encompasses a method for identifying a subject in need of treatment with an inhibitor of RAGE, comprising determining in a sample from said subject the amount of RAGE, comparing said amount of RAGE determined in step a) with a reference amount, and identifying a subject in need of treatment with an inhibitor of RAGE. The invention also relates to a device for identifying a subject in need of treatment with an inhibitor of RAGE, as well as a kit adapted to carry out the method of the present invention, said kit comprising instructions for carrying out the aforementioned method.

The Receptor for Advanced Glycation-End products (RAGE) is a type I transmembrane receptor belonging to the immunoglobulin-like superfamily. Originally identified as receptor for advanced glycation end-products (AGEs), Rage is nowadays considered as a pattern-recognition receptor, given its ability to bind different ligands such as high mobility group box 1 (HMGB 1), several members of the S100 protein family and amyloid β peptides (Boyd et al., 2008; Ghavami et al., 2008; Sims et al., 2010). Under physiological conditions, RAGE is constitutively expressed at high levels only in the lung (Fehrenbach et al., 1998), while other tissues display low expression levels mainly on vascular endothelial cells, dentritic cells, neutrophils, monocytes/macrophages, lymphocytes, neurons and cardiomyocytes (Sims et al., 2010).

RAGE binding to its ligands promotes the activation of pro-inflammatory responses and, in a feed forward loop, increases the expression of the receptor itself. As a consequence, RAGE has been shown to play an important role in different acute and chronic inflammatory diseases, in septic shock and in late diabetic complications (reviewed by Bierhaus and Nawroth, 2009; Riehl et al., 2009).

Besides inflammation, a strong upregulation of RAGE and its ligands has been shown in different cancer entities, and numerous experimental studies support a critical role in tumorigenesis (Riehl et al., 2009; Sims et al., 2010, Logsdon et al., 2007). In particular, blockade of HMGB1-RAGE interaction resulted in decreased tumor growth and metastasis in a mouse xenograft model (Taguchi et al., 2000). Moreover, recent findings highlighted a crucial role for RAGE in inflammation-driven skin carcinogenesis and colitis-associated cancer (Gebhardt et al., 2008; Turovskaya et al., 2008). In these settings Rage-deficient mice displayed impairment in inflammatory cell recruitment and cytokine production during the initial phase of tumor promotion, implicating that RAGE represents a key player in the establishment of a pro-inflammatory environment and in the communication between tumor and immune cells (Riehl et al., 2009).

In the liver, several reports demonstrated that the HMGB1-RAGE axis plays a critical role in injury and inflammation. Indeed, RAGE blockade was shown to exert beneficial effects, increasing survival and decreasing liver damage, necrosis and fibrosis in mouse models of ischemia/reperfusion (I/R), partial hepatectomy, acetaminophen-induced liver damage and in a rat model of carbon tetrachloride-induced hepatic fibrosis (Cataldegirmen et al., 2005; Ekong et al., 2006; Zeng et al., 2009; Zeng et al., 2004). Moreover, HMGB1 cytoplasmic relocation in hepatocytes and increased HMGB1 serum levels were observed in mouse models of I/R and in patients with liver failure and chronic hepatitis B infection (Albayrak et al., 2010; Evankovich et al., 2010; Tsung et al., 2005; Xia et al., 2008; Zhou et al., 2011). Finally, increased levels of RAGE and HMGB1 have been shown in hepatocellular carcinomas (HCCs), suggesting that RAGE signaling could play an important role in HCC development (Hiwatashi et al., 2008; Jiang et al., 2011; Kostova et al., 2010). However, our knowledge on the underlying molecular mechanism how RAGE signaling contributes to the pathogenesis of HCC is limited, given the fact that liver cell compartments expressing RAGE and which are directly affected by its blockade still remain controversial (reviewed by Basta et al., 2011).

Despite the aforementioned elucidation of molecular pathways in connection with RAGE resulting in cancer, measures for efficiently treating and/or preventing inflammation- and/or damage-induced cancer are not yet available but nevertheless highly desired.

The technical problem underlying the present invention, therefore, can be seen as the provision of means and methods for complying with the aforementioned needs. The technical problem is solved by the embodiments characterized in the claims and herein below.

Accordingly, the present invention relates to an inhibitor of the Receptor for Advanced Glycation-End products (RAGE) for use in treating and/or preventing inflammation- and/or damage-induced cancer.

The term "Receptor for Advanced Glycation-End products (RAGE)" refers to a type I transmembrane receptor belonging to the immunoglobulin-like superfamily. Originally identified as receptor for advanced glycation end-products (AGEs), RAGE is nowadays considered as a pattern-recognition receptor, given its ability to bind different ligands such as high mobility group box 1 (HMGB1), several members of the S100 protein family and amyloid β peptides (Boyd et al., 2008; Ghavami et al., 2008; Sims et al., 2010). Under physiological conditions, RAGE is constitutively expressed at high levels only in the lung (Fehrenbach et al., 1998), while other tissues display low expression levels mainly on vascular endothelial cells, dentritic cells, neutrophils, monocytes/macrophages, lymphocytes, neurons and cardiomyocytes (Sims et al., 2010). Upon ligand binding, RAGE exhibits biological activity in that pro-inflammatory responses become activated and/or, in that, in a feed forward loop, the expression of the receptor itself is increased. As a consequence, RAGE has been shown to play an important role in different acute and chronic inflammatory diseases, in septic shock and in late diabetic complications (reviewed by Bierhaus and Nawroth, 2009; Riehl et al., 2009). Besides inflammation, a strong upregulation of RAGE and its ligands has been shown in different cancer entities, and numerous experimental studies support a critical role in tumorigenesis (Riehl et al., 2009; Sims et al., 2010). The amino acid and nucleic acid sequences of human RAGE have been described in Neeper et al., 1992. Preferably, the RAGE referred to herein is one of the isoforms of human RAGE encoded by the human RAGE gene (Genbank Acc. No.: NC_000006.11 GI:224589818, SEQ ID NO: 3). More preferably, the RAGE referred to herein is having an amino acid sequence as shown in GenBank Acc. No. : NP_001193858.1 GI:332800965 (SEQ ID NO: 2) or being encoded by a nucleic acid sequence as shown in Genbank Acc. No.: NM_001206929.1 GI:332800964 (SEQ ID NO: 1).

Moreover, the term encompasses variants of RAGE and, in particular, the aforementioned human RAGE. Such variants have at least the same essential biological and immunological properties as the specific RAGE polypeptide. Variants are deemed to share the same essential biological and immunological properties if they are detectable by the same specific assays referred to in this specification, e.g., by ELISA assays using polyclonal or monoclonal antibodies specifically recognizing the said RAGE polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific RAGE polypeptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific RAGE polypeptides or the aforementioned types of variants as long as these fragments have the essential immunological and biological properties as referred to above. Such fragments may be, e.g., degradation products or splice variants of the RAGE polypeptides. Further included are variants which differ due to posttranslational modifications such as phosphorylation, glycosylation, ubiquitinylation, sumoylation or myristylation.

The term "inhibitor of RAGE" encompasses compounds which are capable of specifically inhibiting at least one biological activity of RAGE. Biological activities of RAGE which are preferably inhibited by the inhibitor according to the invention are specified above. The inhibitor may directly or indirectly inhibit the at least one biological activity of RAGE.

A direct inhibition is achieved by a compound which binds to RAGE and thereby inhibits the said biological activity. Compounds which directly inhibit RAGE in this sense are, preferably, compounds which block the interaction of RAGE with other proteins or with its ligands. Alternatively, but nevertheless preferred a direct inhibitor of RAGE may induce an allosteric change in the conformation of the RAGE polypeptide. The allosteric change may subsequently block the interaction of RAGE with other proteins or with its ligands and, thus, interfere with the biological activity of RAGE. Compounds which are suitable as direct inhibitors of RAGE encompass antibodies, aptameres, soluble mutants or variants of the RAGE polypeptide, a dominant-negative high mobility group box 1 protein (HMGB1), and a small molecule antagonist.

Accordingly, the inhibitor of the present invention is, preferably, an antibody which specifically binds to RAGE and inhibits RAGE activity. More preferably, the said antibody when bound to RAGE prevents the binding of a RAGE ligand. Most preferably, said antibody is an anti-RAGE antibody as described in Buttari et al., 2011, Saha et al., 2010, Ghavami et al., 2008a, or by Bierhaus et al., 2004.

The term "antibody" as used herein encompass to all types of antibodies which, preferably, specifically binds to RAGE and inhibits RAGE activity. Preferably, the antibody of the present invention is a monoclonal antibody, a polyclonal antibody, a single chain antibody, a chimeric antibody or any fragment or derivative of such antibodies being still capable of binding to RAGE and inhibiting at least one of its biological activities. Such fragments and derivatives comprised by the term antibody as used herein encompass a bispecific antibody, a synthetic antibody, an Fab, F(ab)₂ Fv or scFv fragment, or a chemically modified derivative of any of these antibodies. Specific binding as used in the context of the antibody of the present invention means that the antibody does not cross-react with other polypeptides. Specific binding can be tested by various well known techniques. Preferably, specific binding can be tested as described in the accompanying Examples. Antibodies or fragments thereof, in general, can be obtained by using methods which are described, e.g., in Harlow and Lane (1988). Monoclonal antibodies can be prepared by the techniques which comprise the fusion of mouse myeloma cells to spleen cells derived from immunized mammals and, preferably, immunized mice (Köhler (1975) and Galfré (1981)). Preferably, an immunogenic peptide having the extracellular domain of RAGE is applied to a mammal. The said peptide is, preferably, conjugated to a carrier protein, such as bovine serum albumin, thyroglobulin, and keyhole limpet hemocyanin (KLH). Depending on the host species, various adjuvants can be used to increase the immunological response. Such adjuvants encompass, preferably, Freund's adjuvant, mineral gels, e.g., aluminum hydroxide, and surface active substances, e.g., lysolecithin, pluronic polyols, polyanions, peptides, oil emulsions, keyhole limpet hemocyanin, and dinitrophenol. Monoclonal antibodies which specifically bind to RAGE can be subsequently prepared using the well known hybridoma technique, the human B cell hybridoma technique, and the EBV hybridoma technique. Specifically binding antibodies which affect at least one biological activity of RAGE can be identified by assays known in the art and described, e.g., in the accompanying Examples, below.

Moreover, the inhibitor of the present invention is, preferably, an aptamere which specifically binds to RAGE and inhibit RAGE activity.

The term "aptamere" as used herein are oligonucleic acid or peptide molecules that bind to a specific target polypeptide (Ellington (1990), Bock (1992)). Oligonucleic acid aptamers are engineered through repeated rounds of selection or the so called systematic evolution of ligands by exponential enrichment (SELEX technology). Peptide aptamers are designed to interfere with protein interactions inside cells. They usually comprise of a variable peptide loop attached at both ends to a protein scaffold. This double structural constraint shall increase the binding affinity of the peptide aptamer into the nanomolar range. Said variable peptide loop length is, preferably, composed of ten to twenty amino acids, and the scaffold may be any protein having improved solubility and compacity properties, such as thioredoxin-A. Peptide aptamer selection can be made using different systems including, e.g., the yeast two-hybrid system (see e.g., Hoppe-Seyler (2000)). Aptameres which affect at least one biological activity of RAGE can be identified by functional assays known in the art.

Further, the inhibitor of the present invention is, preferably, a soluble RAGE polypeptide.

The term "soluble RAGE polypeptide" as used herein refers to a decoy receptor derived from RAGE as described by Frommhold et al., 2010.

Furthermore, the inhibitor of the present invention is, preferably, a dominant-negative high mobility group box 1 protein (HMGB1).

The term "dominant-negative high mobility group box 1 protein (HMGB1)" as used herein refers to a fragment of the HMGB1 polypeptide which lacks the capability of RAGE activation but which is able to prevent the activation of RAGE elicited by unmodified HMGB1. Such a dominant negative HMBG1 is the box A fragment of HMBG1 as described, e.g., in Palumbo et al., 2004, Yang et al., 2004, Dumitriu et al., 2005, and Sitia et al., 2007.

Moreover, the inhibitor of the present invention is, preferably, a small molecule antagonist.

The term "small molecule antagonist" as used herein refers to a chemical compound that specifically interacts and inhibits RAGE. A small molecule as used herein preferably has a molecular weight of less than 1000 Da, more preferably, less than 800 Da, less tha 500 Da, less than 300 Da, or less than 200 Da. Such small molecules are capable of diffusing across cell membranes so that they can enter and reach intracellular sites of action. Suitable chemical compounds encompass small organic molecules. Suitable classes for such small organic molecules may, preferably, be isoprenoids, steroids, flavonoids, glycosides, alkaloids, lipids, alcohols, phenazines, phenols, polyketides, terpenes, or tetrapyrroles.

In principle, a RAGE inhibitor according to the present invention can be identified by analyzing oval cell activation in test animals, such as mice, wherein the test animals have been fed with Choline Deficient Ethionine supplemented (CDE) diet in the presence of the compound suspected to be an RAGE inhibitor. If no activation is detectable for oval cells isolated from livers of animals treated with the compound suspected to be an inhibitor of RAGE, this is an indication for the compound being an inhibitor of RAGE. How such an assay for determining an inhibitor of RAGE can be carried out is described in the accompanying Examples, below.

Inhibition of RAGE according to the present invention can also be achieved by indirect inhibition wherein the amount of RAGE molecules in a cell is reduced. Such a reduction of RAGE molecules is, preferably, accomplished by a reduction or prevention of the expression of the RAGE gene, i.e. by a reduction or prevention of transcription, a destabilization or increased degradation of the transcripts or a reduction or prevention of the translation of the transcripts into RAGE polypeptides. Compounds which are known to interfere with transcription and/or translation of genes as well as stability of transcripts are inhibitory nucleic acids. Such inhibitory nucleic acids, usually, recognize their target transcripts by hybridization of nucleic acid sequences present in both, the target transcript and the inhibitory nucleic acid, being complementary to each other. Accordingly, for a given transcript with a known nucleic acid sequence, such inhibitors can be designed and synthesized without further ado by the skilled artisan. Suitable assays for testing the activity are known in the art and described herein below in the Examples. Specifically, the presence or absence of the target transcript can be measured or the presence or absence of the protein encoded thereby can be measured in the presence and absence of the putative inhibitory nucleic acid. A nucleic acid which, indeed, is an inhibitory nucleic acid can be subsequently identified if in the presence of the inhibitory nucleic acid, the target transcript or the polypeptide encoded thereby can no longer be detected or is detectable at reduced amounts. Alternatively, the biological activities of RAGE can be analyzed as described in detail in the Examples, below.

Accordingly, the inhibitor of the invention is, preferably, an inhibitory nucleic acid. More preferably, said inhibitory nucleic acid is selected from the group consisting of: an antisense RNA, a ribozyme, a siRNA, a micro RNA, a morpholino or a triple helix forming agent.

The term "antisense RNA" as used herein refers to RNA which comprise a nucleic acid sequence which is essentially or perfectly complementary to the target transcript. Preferably, an antisense nucleic acid molecule essentially consists of a nucleic acid sequence being complementary to at least 100 contiguous nucleotides, more preferably, at least 200, at least 300, at least 400 or at least 500 contiguous nucleotides of the target transcript. How to generate and use antisense nucleic acid molecules is well known in the art (see, e.g., Weiss, 1997).

The term "ribozyme" as used herein refers to catalytic RNA molecules possessing a well defined tertiary structure that allows for catalyzing either the hydrolysis of one of their own phosphodiester bonds (self-cleaving ribozymes), or the hydrolysis of bonds in other RNAs, but they have also been found to catalyze the aminotransferase activity of the ribosome. The ribozymes envisaged in accordance with the present invention are, preferably, those which specifically hydrolyse the target transcripts. In particular, hammerhead ribozymes are preferred in accordance with the present invention. How to generate and use such ribozymes is well known in the art (see, e.g., Hean and Weinberg, 2008).

The term "siRNA" as used herein refers to small interfering RNAs (siRNAs) which are complementary to target RNAs (encoding a gene of interest) and diminish or abolish gene expression by RNA interference (RNAi). Without being bound by theory, RNAi is generally used to silence expression of a gene of interest by targeting mRNA. Briefly, the process of RNAi in the cell is initiated by double stranded RNAs (dsRNAs) which are cleaved by a ribonuclease, thus producing siRNA duplexes. The siRNA binds to another intracellular enzyme complex which is thereby activated to target whatever mRNA molecules are homologous (or complementary) to the siRNA sequence. The function of the complex is to target the homologous mRNA molecule through base pairing interactions between one of the siRNA strands and the target mRNA. The mRNA is then cleaved approximately 12 nucleotides from the 3' terminus of the siRNA and degraded. In this manner, specific mRNAs can be targeted and degraded, thereby resulting in a loss of protein expression from the targeted mRNA. A complementary nucleotide sequence as used herein refers to the region on the RNA strand that is complementary to an RNA transcript of a portion of the target gene. The term "dsRNA" refers to RNA having a duplex structure comprising two complementary and anti-parallel nucleic acid strands. Not all nucleotides of a dsRNA necessarily exhibit complete Watson-Crick base pairs; the two RNA strands may be substantially complementary. The RNA strands forming the dsRNA may have the same or a different number of nucleotides, with the maximum number of base pairs being the number of nucleotides in the shortest strand of the dsRNA. Preferably, the dsRNA is no more than 49, more preferably less than 25, and most preferably between 19 and 23, nucleotides in length. dsRNAs of this length are particularly efficient in inhibiting the expression of the target gene using RNAi techniques. dsRNAs are subsequently degraded by a ribonuclease enzyme into short interfering RNAs (siRNAs). The complementary regions of the siRNA allow sufficient hybridization of the siRNA to the target RNA and thus mediate RNAi. In mammalian cells, siRNAs are approximately 21-25 nucleotides in length. The siRNA sequence needs to be of sufficient length to bring the siRNA and target RNA together through complementary base-pairing interactions. The siRNA used with the Tet expression system of the invention may be of varying lengths. The length of the siRNA is preferably greater than or equal to ten nucleotides and of sufficient length to stably interact with the target RNA; specifically 15-30 nucleotides; more specifically any integer between 15 and 30 nucleotides, most preferably 15, 16, 17, 18, 19, 20, 21, 22, 23, 24, 25, 26, 27, 28, 29, and 30. By "sufficient length" is meant an oligonucleotide of greater than or equal to 15 nucleotides that is of a length great enough to provide the intended function under the expected condition. By "stably interact" is meant interaction of the small interfering RNA with target nucleic acid (e.g., by forming hydrogen bonds with complementary nucleotides in the target under physiological conditions). Generally, such complementarity is 100% between the siRNA and the RNA target, but can be less if desired, preferably 91%, 92%, 93%, 94%, 95%, 96%, 97%, 98%, or 99%. For example, 19 bases out of 21 bases may be base-paired. In some instances, where selection between various allelic variants is desired, 100% complementary to the target gene is required in order to effectively discern the target sequence from the other allelic sequence. When selecting between allelic targets, choice of length is also an important factor because it is the other factor involved in the percent complementary and the ability to differentiate between allelic differences. Methods relating to the use of RNAi to silence genes in organisms, including C. elegans, Drosophila, plants, and mammals, are known in the art (see, for example, Fire, 1998; Fire, 1999; Sharp, 2001; Hammond, 2001; Tuschl, 2001; Hamilton, 1999; Hammond, 2000; Zamore, 2000; Bernstein, 2001; Elbashir, 2001; WO 0129058; WO 09932619; and Elbashir, 2001a).

The term "microRNA" as used herein refers to a self complementary single-stranded RNA which comprises a sense and an antisense strand linked via a hairpin structure. The micro RNA comprise a strand which is complementary to an RNA targeting sequences comprised by a transcript to be downregulated. micro RNAs are processed into smaller single stranded RNAs and, therefore, presumably also act via the RNAi mechanisms. How to design and to synthesise microRNAs which specifically degrade a transcript of interest is known in the art and described, e.g., in EP 1 504 126 A2 or Dimond, 2010.

The term "morpholino" refers to a synthetic nucleic acid molecule having a length of 20 to 30 nucleotides, preferably, about 25 nucleotides. Morpholinos bind to complementary sequences of target transcripts by standard nucleic acid base-pairing. They have standard nucleic acid bases which are bound to morpholine rings instead of deoxyribose rings and linked through phosphorodiamidate groups instead of phosphates (see, e.g., Summerton (1997)). The replacement of anionic phosphates with the uncharged phosphorodiamidate groups eliminates ionization in the usual physiological pH range, so morpholinos in organisms or cells are uncharged molecules. The entire backbone of a morpholino is made from these modified subunits. Unlike inhibitory small RNA molecules, morpholinos do not degrade their target RNA molecules. Rather, they sterically block binding to a target sequence within an RNA and simply getting in the way of molecules that might otherwise interact with the RNA (see, e.g., Summerton, 1999).

The term "triple helix forming agent" as used herein refers to oligonucleotiges which are capable of forming a triple helix with DNA and, in particular, which interfere upon forming of the triple-helix with transcription initiation or elongation of a desired target gene such as RAGE in the case of the inhibitor of the present invention. The design and manufacture of triple helix forming agents is well known in the art (see, e.g., Vasquez, 2002).

The term "inflammation- and/or damage-induced cancer" as used herein refers to cancer which is caused by or promoted by inflammatory responses or tissue damage. Preferably, said inflammatory response is a chronic inflammatory response, i.e. chronic inflammation. Also preferably, said inflammation- and/or damage-induced cancer is hepatocarcinoma or hepatocellular carcinoma, cholangiocarcinoma, pancreas cancer, gastrointestinal and colon cancer, esophageal carcinoma.

The inhibitor according to the present invention shall be provided for use in treating inflammation- and/or damage-induced cancer, i.e. it shall be manufactured as a medicament, e.g., comprised in a pharmaceutical composition. Details on such a medicament or pharmaceutical composition are to be found elsewhere herein. The inhibitor of RAGE to be used according to the present invention can also be applied in a method for treating or preventing inflammation- and/or damage-induced cancer. Accordingly, the invention also contemplates a method for treating or preventing inflammation- and/or damage-induced cancer in a subject suffering therefrom comprising administering to said subject a therapeutically effective amount of an inhibitor of RAGE as defined herein.

Advantageously, it has been found in the studies underlying the present invention that RAGE supports hepatocellular carcinoma (HCC) formation in a model of inflammation-driven tumorigenesis, the *multidrug resistance* 2 knock-out (*Mdr2*-/-) mouse, while it is dispensable upon treatment with the carcinogen diethynitrosammine. In *Mdr2-*/*-* mice RAGE ablation reduces tissue damage in the pre-neoplastic liver accompanied by severely impaired activation of hepatic progenitor cells, the so called oval cells. Indeed, oval cells express high levels of RAGE and exhibit increased proliferation upon treatment with the RAGE ligand high mobility group box 1 (HMGB 1). Finally, impaired oval cell activation upon RAGE blockade in vitro and in vivo demonstrates that RAGE represents a drug target for the prevention and therapy of inflammation- and damage-driven hepatocarcinogenesis.

More specifically, in the liver, RAGE has been reported to play an important role in injury and inflammatory states. Indeed, RAGE signaling blockade improved the outcome in several mouse models of hepatic injury, increasing survival and decreasing necrosis and fibrosis (Cataldegirmen et al., 2005; Ekong et al., 2006; Zeng et al., 2009; Zeng et al., 2004). Since hepatic damage is a prerequisite to HCC formation (Block et al., 2003), it was hypothesized that Rage expression could directly affect hepatocarcinogenesis. To verify this assumption, the consequence of RAGE ablation (Rage-/-) in two opposite HCC models was analyzed: the hepatocarcinogen DEN, a model that relies mainly on mutations induced in hepatocytes and is devoid of cirrhosis (Chakraborty et al., 2007; Grivennikov et al., 2010; Tatematsu et al., 1988; Vainer et al., 2008), and the Mdr2-/- model in which chronic injury and hepatitis drive tumorigenesis (Mauad et al., 1994; Pikarsky et al., 2004). Interestingly, RAGE ablation significantly impaired HCCs formation in the Mdr2-/- mouse, whereas Rage-/-DEN mice displayed lesions that were comparable in number and size to wtDEN but were slightly enriched for dysplastic nodules. This indicates that in the liver Rage expression per se is not sufficient to induce hepatocarcinogenesis but becomes necessary in settings of chronic injury and inflammation. In line with this assumption, pre-malignant DENwt and DENRage-/- mice did not show clear signs of inflammation and injury 6 months after injection. On the contrary, pre-malignant Mdr2-/- and Mdr2-/-Rage-/- (dKO) mice displayed evidence of chronic liver damage, inflammatory infiltrates and fibrotic deposition. Importantly, these features recapitulate all key traits of human HCC development. Furthermore, only two dKO mice exhibited one single HCC and lesions were mainly classified as premalignant dysplastic nodules. The comparable percentage of lesion-free mice between Mdr2-/- and dKO livers suggests that Rage deficiency delays the onset and establishment of malignant transformation, further highlighting the role played by Rage in the malignant progression of liver tumors. Similarly, Rage-/- mice developed chemically induced skin tumors that were smaller in size, less progressed and highly differentiated compared to wild type controls (Gebhardt et al., 2008). RAGE is highly expressed on leukocytes and endothelial cells and its engagement by HMGB 1, S 100 proteins and AGEs critically contributes to acute and chronic inflammatory responses (Sims et al., 2010). Hence, deletion of RAGE hampered the recruitment of inflammatory cells or the secretion of pro-inflammatory cytokines in mouse models of inflammation-induced skin and colon carcinogenesis (Gebhardt et al., 2008; Turovskaya et al., 2008). In contrast to these chemically induced tumor models, no significant impairment in the recruitment of inflammatory cells in dKO compared to Mdr2-/- mice was detected. This may be due, at least in part, to compensatory signaling initiated by other damage associated molecular pattern (DAMP) receptors such as TLR4, which has been shown to play a crucial role in hepatitis (Pradere et al., 2010). A further possibility is that ICAM-1 may compensate for the absence of RAGE (Frommhold et al., 2011; Frommhold et al., 2010). Moreover, it cannot be exclude that the impact of RAGE on leukocyte infiltration and establishment of an inflammatory microenvironment critically depends on the initial cause and chronological sequence of tissue activation either by chemical agents or altered physiology due to Mdr2 deletion. Chronic liver damage fosters HCC development and several studies support an involvement of RAGE in the pathogenesis of liver damage accompanied by cirrhosis, non-alcoholic steatohepatitis (NASH), and cancer (Hyogo and Yamagishi, 2008). Indeed, it could be demonstrate that RAGE ablation in Mdr2-/- mice significantly reduces liver damage and fibrosis. Although this is in line with previous reports (Ekong et al., 2006; Zeng et al., 2004), the underlying molecular mechanism and critical cells that express RAGE in the liver under pathological conditions remain elusive. In cases of chronic and severe liver damage, oval cells (hepatic progenitor cells) get activated, expand and invade the liver parenchyma from the portal triad, sustaining liver regeneration and restoring liver homeostasis (Sell and Leffert, 2008). However, chronic oval cell activation was evident in the pre-malignant phase both in a model of lymphotoxin-driven liver tumorigenesis (Haybaeck et al., 2009) and in a model of non-alcoholic steato-hepatitis (de Lima et al., 2008). Accordingly, impaired liver tumorigenesis in the TNF receptor type 1 knockout mouse was associated with reduced oval cell activation (Knight et al., 2000). Although a causal role of oval cells in HCC development has not been addressed yet, since oval cell proliferation is usually observed during the preneoplastic stages of liver tumorigenesis, it can be assumed that activation of the oval cell compartment in the chronic injured liver would correlate with the possibility of HCC development.

In the studies underlying this invention, a severe impairment in oval cell activation in dKO compared to Mdr2-/- livers during the pre-neoplastic phase was found. Notably, DEN-treated mice which displayed only mild levels of sustained liver injury, were devoid of oval cell activation during the pre-malignant phase and displayed an almost absent activation during malignancy. On the contrary, oval cells were extremely activated in the liver parenchyma of Mdr2-/- mice. When RAGE expression in immune cells, oval cells and hepatocytes isolated from CDE livers was analyzed, it was at its highest in oval cells, further suggesting a direct role for RAGE in this cell compartment. Although, the liver cell compartments expressing RAGE still remain controversial, in line with previous reports, we detected an almost absent expression of Rage in hepatocytes (reviewed by Basta et al., 2011). This observation, taken together with the fact that RAGE ablation does not affect HCC upon DEN treatment, implies that RAGE does not exert a direct role when HCC formation is induced by DNA mutations in hepatocytes. Evidence from the Mdr2-/- model instead indicates that Rage expression in a chronic inflamed liver and, in particular on oval cells, might sustain tumor formation by activating oval cells and fostering damage and vicious cycles of death and regeneration. In line with this assumption, silencing of RAGE expression in vitro dramatically decreased growth of the BMOL oval cell line and treatment with the Rage ligand HMGB1 promoted BMOL cell growth and induced activation of an ERK1/2-cyclin D1 signaling cascade (Guo et al., 2011; Ranzato et al., 2010), likely explaining the accelerated growth. Several studies demonstrated that the presence of extracellular HMGB1 is causally linked to inflammation and tissue injury (Sims et al., 2010). In Mdr2-/- and dKO livers, infiltrating inflammatory cells were highly positive for HMGB 1, with cytoplasmic HMGB1 relocation, a pre-requisite for its secretion, in the surrounding hepatocytes, whereas in DEN-initiated liver tumors HMGB 1 was exclusively nuclear. Accordingly, HMGB 1 was present at high concentration in the serum of both dKO and Mdr2-/- mice. Cytoplasmic HMGB1 relocation has been associated to increased serum HMGB 1 levels in mouse models of liver injury and to HMGB1 secretion upon LPS and TNF treatment in vitro (Evankovich et al., 2010; Tsung et al., 2005; Zhou et al., 2011). Although HMGB1 levels were comparable in Mdr2-/- and dKO mice, liver damage was significantly decreased in dKO mice. This strongly suggests that inflammation is independent of RAGE, while oval cell activation critically depends on HMGB1-RAGE signaling. Indeed in the CDE model, blockade of RAGE ligands by treatment with the decoy receptor sRAGE or ablation of RAGE, reduced oval cell activation in vivo.

In essence, in rodents, blockade of HMGB1-RAGE interaction has been shown to effectively reduce liver damage only in acute injury (Andrassy et al., 2008; Evankovich et al., 2010; Tsung et al., 2005). Unfortunately, Hmgb1-/- mice die at birth (Calogero et al., 1999) and, hitherto, no conditional Hmgb1 knockout mouse has been established to address the question whether HMGB1 ablation in the Mdr2-/- mouse phenocopies the dKO liver phenotype. Furthermore, it would be of great interest to correlate the expression of RAGE and the abundance of its ligands, in particular HMGB 1, with the severity of disease and its clinical outcome in different human liver disorders. The data demonstrate a crucial role of RAGE signaling in promotion and malignant progression of inflammation and damage-driven HCC. In chronic liver injury settings, the expression of RAGE sustains HCC formation by modifying the liver microenvironment, sustaining damage and promoting oval cell activation. Secretion of HMGB1, a RAGE ligand, promotes liver damage and oval cell proliferation, two risk factors for HCC development. Our results imply that inhibition of RAGE signaling may be considered as a novel strategy for the prevention of HCC development during the early stages of liver injury. Accordingly, by providing the inhibitors of RAGE according to the invention, the prevention and/or treatment of inflammation- and/or damage-induced cancer will greatly benefit.

The explanations and definitions of the terms made above apply for the following embodiments mutatis mutandis.

The invention furthermore relates to a medicament comprising an inhibitor of RAGE as specified above and, preferably, a pharmaceutically acceptable carrier.

The term "medicament" as used herein refers, in one aspect, to a pharmaceutical composition containing the inhibitor referred to above as pharmaceutical active compound, wherein the pharmaceutical composition may be used for human or non-human therapy of various diseases or disorders in a therapeutically effective dose. The inhibitor, preferably, can be present in liquid or lyophilized form. The medicament is, preferably, for topical or systemic administration. Conventionally a medicament will be administered intra-muscular or, subcutaneous. However, depending on the nature and the mode of action of a compound, the medicament may be administered by other routes as well. The inhibitor is the active ingredient of the composition, and is, preferably, administered in conventional dosage forms prepared by combining the drug with standard pharmaceutical carriers according to conventional procedures. These procedures may involve mixing, granulating, and compression, or dissolving the ingredients as appropriate to the desired preparation. It will be appreciated that the form and character of the pharmaceutical acceptable carrier or diluent is dictated by the amount of active ingredient with which it is to be combined, the route of administration, and other well-known variables.

A carrier must be acceptable in the sense of being compatible with the other ingredients of the formulation and being not deleterious to the recipient thereof. The pharmaceutical carrier employed may include a solid, a gel, or a liquid. Examples for solid carriers are lactose, terra alba, sucrose, talc, gelatin, agar, pectin, acacia, magnesium stearate, stearic acid and the like. Exemplary of liquid carriers are phosphate buffered saline solution, syrup, oil, water, emulsions, various types of wetting agents, and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania.

A diluent is selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or non-toxic, non-therapeutic, non-immunogenic stabilizers and the like.

A therapeutically effective dose refers to an amount of the inhibitor to be used in medicament of the present invention which prevents, ameliorates or treats the symptoms accompanying a disease or condition referred to in this specification. Therapeutic efficacy and toxicity of the compound can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen will be determined by the attending physician and other clinical factors. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. The medicament referred to herein is administered at least once in order to treat or ameliorate or prevent a disease or condition recited in this specification. However, the said medicament may be administered more than one time. Specific medicaments are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent. The resulting formulations are to be adapted to the mode of administration. Dosage recommendations shall be indicated in the prescribers or users instructions in order to anticipate dose adjustments depending on the considered recipient.

The medicament according to the present invention may also comprise drugs in addition to the antagonist of the present invention which are added to the medicament during its formulation.

Finally, it is to be understood that the formulation of a medicament takes place under GMP standardized conditions or the like in order to ensure quality, pharmaceutical security, and effectiveness of the medicament.

The invention also relates to a method for identifying a subject in need of treatment with an inhibitor of RAGE and, preferably, an inhibitor as defined above, comprising
a) determining in a sample from said subject the amount of RAGE and / or of at least one marker for inflammation,
b) comparing said amount of RAGE and / or of a marker for inflammation determined in step a) with a reference amount, and
c) identifying a subject in need of treatment with an inhibitor of RAGE.

The term "subject" as referred to herein encompasses animals, preferably mammals, and, more preferably, humans. More preferably, said subject suffers from or is suspected to suffer from inflammation- and/or damage-induced cancer is hepatocarcinoma or hepatocellular carcinoma. Subjects which suffer from the said disease can be identified by the accompanying symptoms known for the diseases. These symptoms are known in the art and described, e.g., in Bruix et al.,2004, Severi et al., 2010, and in El-Serag and Rudolph, 2007. A subject suspect to suffer from the aforementioned disease may be any apparently healthy subject, e.g., investigated by routine clinical screening, or may be a subject being at risk for developing the aforementioned disease. Risk groups for the diseases are known in the art and described in, e.g., Bruix et al., 2004, Severi et al., 2010, and in El-Serag and Rudolph, 2007.

The term "sample" refers to a sample of a body fluid, to a sample of separated cells or to a sample from a tissue or an organ or to a sample of wash/rinse fluid obtained from an outer or inner body surface. Samples can be obtained by well known techniques and include, preferably, scrapes, swabs or biopsies from the digestive tract,liver, pancreas, anal canal, the oral cavity, the upper aerodigestive tract and the epidermis. Such samples can be obtained by use of brushes, (cotton) swabs, spatula, rinse/wash fluids, punch biopsy devices, puncture of cavities with needles or surgical instrumentation. However, samples of blood, plasma, serum, urine, saliva, lacrimal fluid, stool are also encompassed by the method of the present invention. Tissue or organ samples may be obtained from any tissue or organ by, e.g., biopsy or other surgical procedures. Separated cells may be obtained from the body fluids or the tissues or organs by separating techniques such as filtration, centrifugation or cell sorting. Preferably, cell, tissue or organ samples are obtained from those cells, tissues or organs, which are known or suspected to contain RAGE, a RAGE ligand, or markers for inflammation. Preferably, the sample is known or suspected to be comprising RAGE expressing cancer cells, more preferably comprising RAGE expressing oval cells and/or cancer cells or cells which are suspected to be RAGE expressing cancer cells or oval cells. Preferably, the sample is a liver biopsy sample or a serum sample.

The amount of RAGE can be determined in a sample of a subject by techniques well known in the art. Depending on the nature of the sample, the amount may be determined by ELISA based techniques for quantifying the amount of a polypeptide or by quantification of the amount of antibodies in immunohistochemistry of tissue biopsy samples. To this end, antibodies or aptameres may be used as detection agents which specifically bind to RAGE and which upon binding can be detected by a detectable label. How such antibodies or aptameres can be generated is described elsewhere in this specification. A detectable label may be covalently or reversibly linked to the antibody or aptamere. A covalently linked label may be a radioactive, fluorophore or chemiluminescent moiety while a reversible label may be a secondary antibody or an aptamere which specifically binds to the detection agent and which upon binding can be used to detect the bound detection agent. Moreover, the amount of RAGE may also be determined in accordance with the use of the present invention by measures aiming to detect the amount of transcripts of the RAGE gene. Such techniques encompass hybridization techniques such as Northern Blots or PCR-based determination techniques.

As used herein, the term "marker for inflammation" refers to any molecule detectable in a sample whose amount in said sample deviates in a statistically significant way from normal in case there is an inflammation in a subject. Preferably, the marker is produced at the site of inflammation. However, the skilled artisan knows that the marker for inflammation is not necessarily confined to the site of inflammation, but may diffuse or be transported by the blood or lymph system to other regions of the body. Also preferably, the marker may be a secondary marker, the change in concentration of said secondary marker being directly or indirectly affected by a molecule produced at the site of inflammation. Preferably, the marker is a polynucleotide, a protein, or a low-molecular weight molecule. Preferably, the marker for inflammation is a RAGE ligand, e.g. advanced glycation end-product (AGE), High-mobility group protein B1 (HMGB1), members of the S100 protein family, Amyloid-β-protein, Mac-1, or phosphatidylserine. Preferably, the marker for inflammation is a marker for hepatitis or liver damage, e.g. a RAGE ligand as specified above, a serum marker for hepatitis or liver damage, e.g. Alanine transaminase (ALT), Aspartate transaminase (AST), Glutamate dehydrogenase (GLDH), type IV collagen, or a surrogate marker for hepatitis or liver damage, e.g. serum glutamic oxalacetic transaminase, Gamma-GT, type III collagen, and the like. The skilled artisan knows that determination of one of the markers specified herein may not be sufficient to identify a subject suffering from inflammation and, thus, for identifying a subject in need of treatment with an inhibitor of RAGE in a statistically significant way. It is known from the state of the art that the combination of two or more of said markers of inflammation is used to improve statistical significance. It is also known from the state of the art which markers of inflammation are preferably combined.

In accordance with the aforementioned method of the present invention, it will be understood that the amount of RAGE or of a marker of inflammation determined in a sample of a subject should be compared to a reference amount which indicates whether the test subject is in need of the treatment, or not. A suitable reference amount may be derived from a subject or group of subjects known to be in need for the treatment with a RAGE inhibitor. In such a case an amount of the determined RAGE or of the marker of inflammation in the test sample which is identical to the reference amount in the test sample is indicative for a subject in need of the treatment with the RAGE inhibitor. A test amount, which is decreased with respect to the reference amount shall be indicative for a subj ect which is not in need of the said treatment. Alternatively, a suitable reference amount may be derived from a subject or group of subjects known not to be in need of a treatment with a RAGE inhibitor. In such a case an amount of the determined RAGE or of the marker of inflammation in the test sample which is identical with respect to the reference amount in the test sample is indicative for a subject being not in need of the treatment with the RAGE inhibitor while an increased amount is indicative for a subject being in need for the said treatment. The skilled artisan knows that a specific reference amount is to be determined for each of the RAGE or marker of inflammation separately. The skilled artisan also knows that the specific reference amount for a specific RAGE or marker of inflammation may be different for different patient groups. E.g. ALT levels are considered normal in the range 5 - 23 U/l for men and 5 - 19 U/l for women.

The present invention also refers to the general use of RAGE or a detection agent for RAGE in a sample of a subject for identifying a subject in need of treatment with an inhibitor of RAGE.

The invention further provides a device for identifying a subject in need of treatment with an inhibitor of RAGE, comprising a) an analyzing unit comprising a detection agent for determining the amount of RAGE in a sample from said subject and b) an evaluation unit comprising a data processor having tangibly embedded an algorithm for comparing the amount determined by said analyzing unit to a reference amount for said amount of RAGE and which is capable of generating an output file containing a diagnosis established based on said comparison.

The term "device" as used herein relates to a system comprising the aforementioned units operatively linked to each other as to allow the identification according to the methods of the invention. Preferred detection agents, which can be used for the analyzing unit are disclosed elsewhere herein. The analyzing unit, preferably, comprises said detection agents in immobilized form on a solid support which is to be contacted to the sample comprising RAGE the amount of which is to be determined. Moreover, the analyzing unit can also comprise a detector, which determines the amount of detection agent which is specifically bound to RAGE. The determined amount can be transmitted to the evaluation unit. Said evaluation unit comprises a data processing element, such as a computer, with an implemented, tangibly embedded algorithm for carrying out a comparison between the determined amount of RAGE and a suitable reference. Suitable references can be derived from samples of subjects to be used for the generation of reference amounts as described elsewhere herein above. The results may be given as output of parametric diagnostic raw data, preferably, as absolute or relative amounts. It is to be understood that these data will need interpretation by the clinician. However, also envisage are expert system devices wherein the output comprises processed diagnostic raw data the interpretation of which does not require a specialized clinician and may directly serve as an aid for diagnosis.

Finally, the invention relates to a kit adapted to carry out the method of the invention, said kit comprising instructions for carrying out the said method, a detection agent capable of determining the amount of RAGE in a sample and a reference which can be used for comparing the determined amount of RAGE from the sample and allowing identifying a subject in need of treatment with an inhibitor of RAGE.

The term "kit" as used herein refers to a collection of the aforementioned components, preferably, provided in separately or within a single container. The container also comprises instructions for carrying out the method of the present invention. These instructions may be in the form of a manual or may be provided by a computer program code, which is capable of carrying out the comparisons referred to in the methods of the present invention and to establish a diagnosis accordingly when implemented on a computer or a data processing device. The computer program code may be provided on a data storage medium or device such as an optical storage medium (e.g., a Compact Disc) or directly on a computer or data processing device. Moreover, the kit may, preferably, comprise standards for reference amounts as described elsewhere herein in detail.

All references cited in this specification are herewith incorporated by reference with respect to their entire disclosure content and the disclosure content specifically mentioned in this specification.

### FIGURES

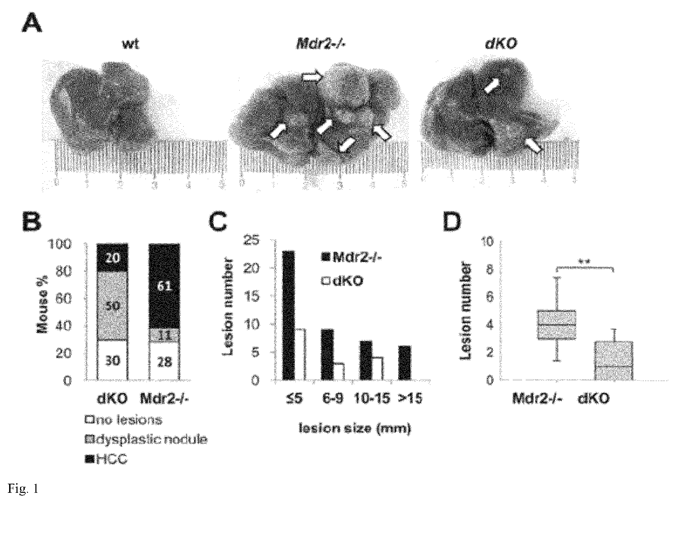

**Figure 1****:** HCC development in *Mdr2*^{-/-} and *dKO* mice. **A** Macroscopy of wt, *Mdr2*^{-/-} and *dKO* mouse livers at 15 months of age (n=10, respectively). Arrows indicate liver lesions and HCCs. **B** Relative distribution of dysplastic nodules and HCCs in *Mdr2*^{-/-} and *dKO* mice (depicted as percentage of lesion bearing mice). **C** Liver lesions divided according to their size (mm) and plotted against their number. **D** Multifocal tumorigenesis per mouse. Error bars represent 5^{th}/95^{th} percentile, **P<0.001.

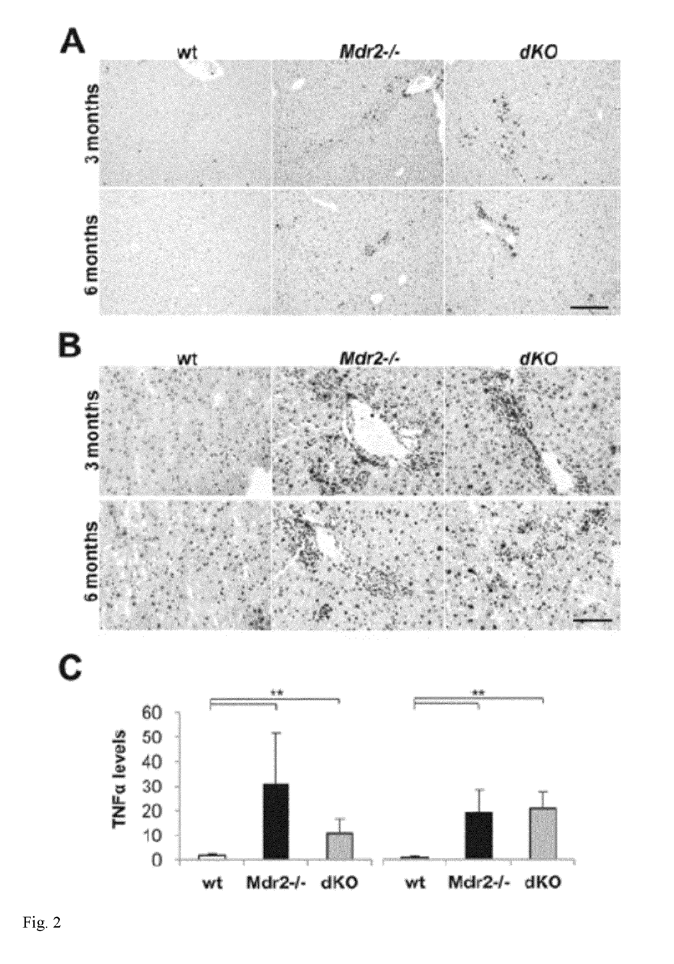

**Figure 2****:** Inflammatory cell recruitment in *Mrd2*^{-/-} and *dKO* livers. **A, B** Immunohistochemical analysis of liver sections from 3 and 6 month old wt, *Mrd2*^{-/-} and *dKO* mice. S100A9 **(A;** bar, 200µm) and myeloperoxidase **(B;** bar, 100µm) stainings (n=5, respectively). **C** TNFα expression analysis. Quantitative RT-PCR for TNFα on cDNA obtained from wt, *Mdr2*^{-/-} and *dKO* liver mRNA (n=5, respectively). Bars represent the mean of TNFα induction normalized to wild type levels at 3 and 6 months of age. Experiments were performed in triplicates; error bar represent SD; **P.0.001.

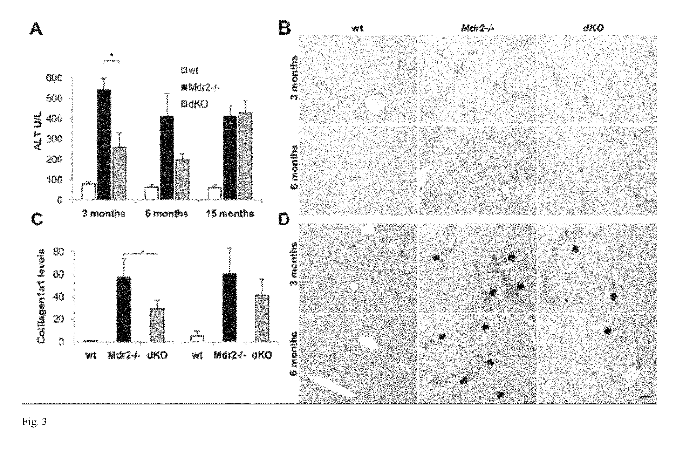

**Figure 3****:** Analysis of liver damage, fibrosis and oval cell activation. **A** ALT serum levels in 3, 6 and 15 month old wt, *Mdr2*^{-/-} and *dKO* mice. Bars represent average ALT serum levels (units/liter) of each mouse group (n=5, respectively); error bars represent SD; *P<0.05. **B** Sirius Red staining of liver sections of 3 and 6 month old mice (n=5, respectively). Bar, 200µm. **C** Collagen1α1 expression levels by quantitative RT-PCR. Bars represent the average Collagen1α1 expression level normalized to wt at 3 and 6 months of age (n=5, respectively). Experiments were performed in triplicates; error bar represent SD; *P< 0.05. **D** Analysis of oval cell activation. Staining for the oval cell specific A6 antigen on liver sections from 3 and 6 month old wt, *Mdr2*^{-/-} and *dKO* mice (n=5, respectively). Black arrows indicate A6 positive staining; bar, 200µm.

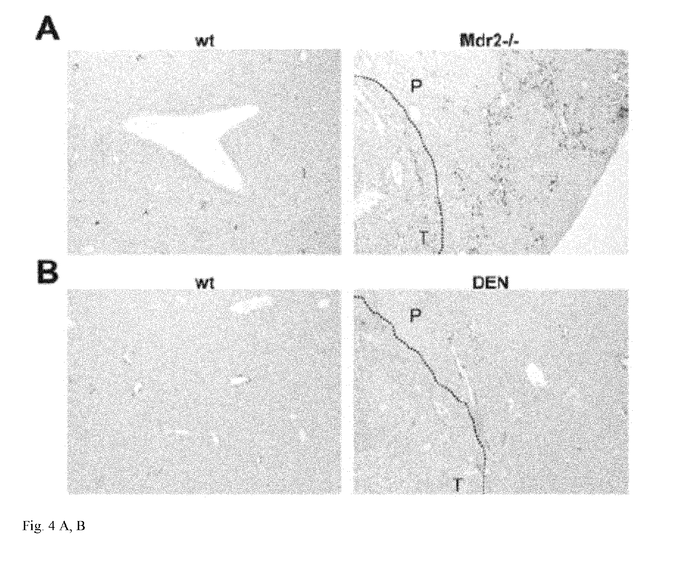

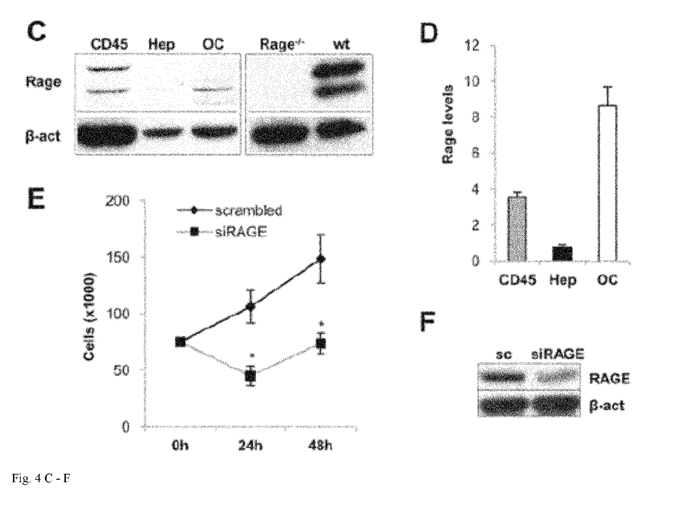

**Figure 4****:** Rage expression in oval cells. **A, B** Analysis of oval cell activation in *Mdr2*^{-/-} and DEN-derived tumor parenchyma. Staining with anti-A6 antibody of liver sections from: **A** 15 month-old *Mdr2*^{-/-} and age-matched wt mouse (n=10), **B** DEN- and NaCl-injected mouse 12 months upon injection (n=8). Black dashed line divides the tumor (T) from the liver parenchyma (P). Western blot **(C)** and quantitative RT-PCR **(D)** for Rage on hepatocytes (hep), oval (OC) and inflammatory (CD45) cells isolated from CDE-treated mice. **C** Wt and *Rage*^{-/-} lung lysates served as positive and negative controls, anti-β-actin antibody as loading control. Experiments were repeated 3 times. **D** Bars represent the average Rage expression level normalized to the hepatocyte fraction. Experiments were performed in triplicates. **E** BMOL cells transfected with scrambled and siRAGE oligonucleotides, starved and treated with 2% FCS medium. The graph represents the number of cells after 24 and 48 hours of stimulation; experiments were repeated 3 times in duplicate; *P<0.05. **F** Western blot for Rage on protein lysates obtained from BMOL cells transfected with siRAGE or scrambled (sc) oligonucleotides. Anti-β-actin antibody was used as loading control. Experiments were repeated 3 times.

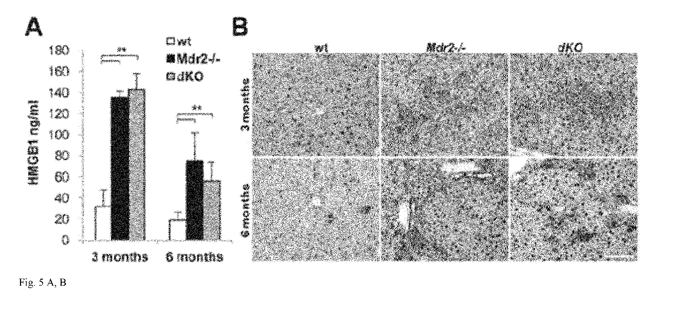

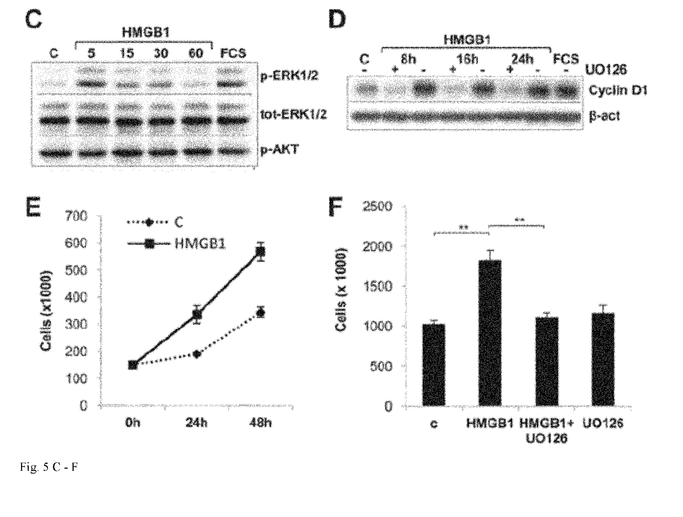

**Figure 5****:** Role of HMGB1 in oval cell activation. **A** HMGB1 ELISA. HMGB1 serum levels (ng/ml) of 3 and 6 month old wt, *Mdr2*^{-/-} and *dKO* mice (n=5, respectively); **P<0.001. **B** Immunohistochemical staining for HMGB1 on liver sections of 3 and 6 month old wt, *Mdr2*^{-/-}and *dKO* mice (n=5, respectively; bar, 200µm). **C** Western blot on protein lysates obtained from BMOL cells treated with serum-free medium (C), 2% FCS medium (FCS) and 30 ng/ml HMGB1 (for the indicated minutes). Antibodies: anti-phospho-ERK1/2, total ERK1/2 and anti-phospho-AKT. Experiments were repeated 3 times. **D** Western blot for anti-cyclin D1 and β-action antibodies on protein lysates obtained from BMOL cells treated with serum-free (C), 2% FCS medium (FCS) and 30 ng/ml HMGB1 (for the indicated hours). BMOLs were pretreated with 10 µM UO126 as indicated. Experiments were repeated 3 times. **E** HMGB1-induced proliferation. Starved BMOL cells were treated with serum-free medium alone (C) or containing HMGB1 (30 ng/ml). The graph represents the number of cells after 24 and 48 hours of stimulation. Experiments were repeated 3 times in duplicate, error bars represent SD. **F** Inhibition of HMGB1-induced proliferation. Starved BMOL cells were treated with serum-free medium (C), HMGB1 (30 ng/ml) with or without 10 µM UO126 or UO126 alone. The graph represents the number of cells after 48 hours of stimulation; experiments were repeated 3 times in duplicate. Error bar represent SD; **P< 0.001.

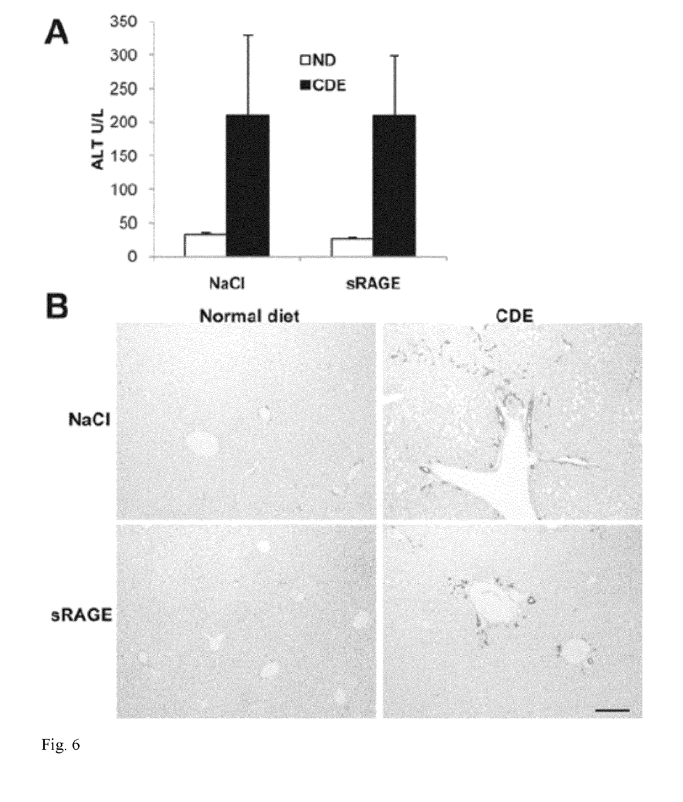

**Figure 6****:** sRAGE treatment of CDE fed mice. **A** Analysis of ALT serum levels in wt mice fed a normal (ND) or a choline deficient ethionine-supplemented (CDE) diet, treated with NaCl or sRAGE (100µg/mouse). Bars represent average ALT serum levels (units/liter) of each mouse group (n=5, respectively); error bars represent SD. **B** Analysis of oval cell activation. Staining for the A6 antigen on liver sections from wt mice fed a normal or a choline deficient ethionine-supplemented (CDE) diet, treated with NaCl or sRAGE (100/µg/mouse); bar, 200µm.

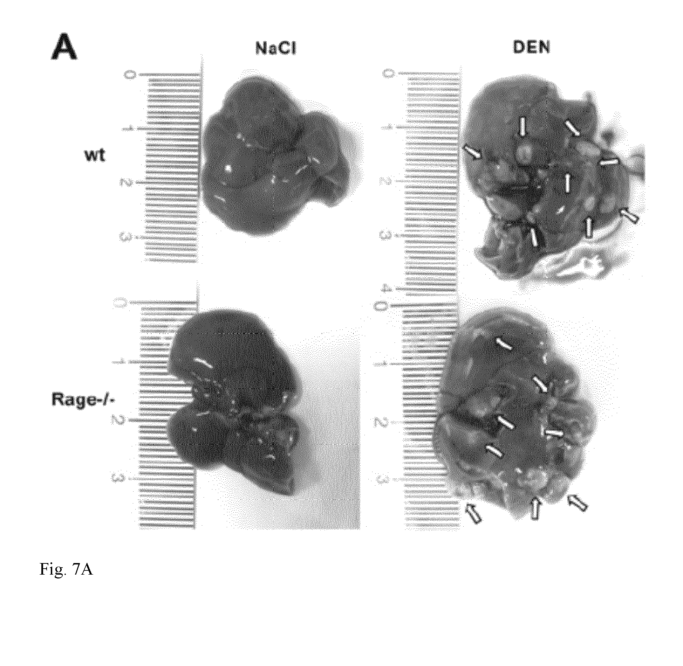

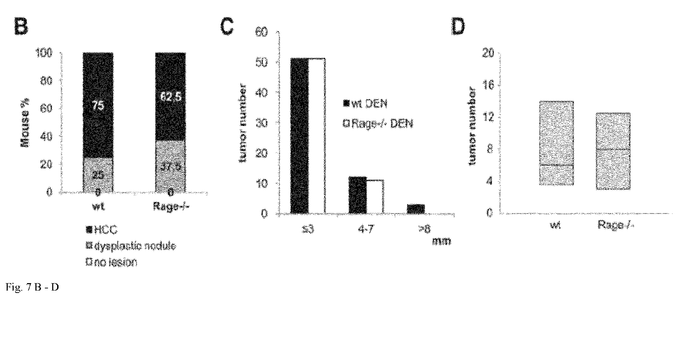

**Figure 7****:** HCC development in DEN-treated wt and *Rage*^{-/-} mice. **A** Macroscopy of wt and *Rage*^{-/-} mouse livers 12 month upon DEN injection (n=8, respectively). Arrows indicate liver lesions and HCCs. **B** Relative distribution of dysplastic nodules and HCCs in wt and *Rage*^{-/-}DEN-treated mice (depicted as percentage of lesion bearing mice). **C** Liver lesions divided according to their size (mm) and plotted against their number. **D** Multifocal tumorigenesis per mouse.

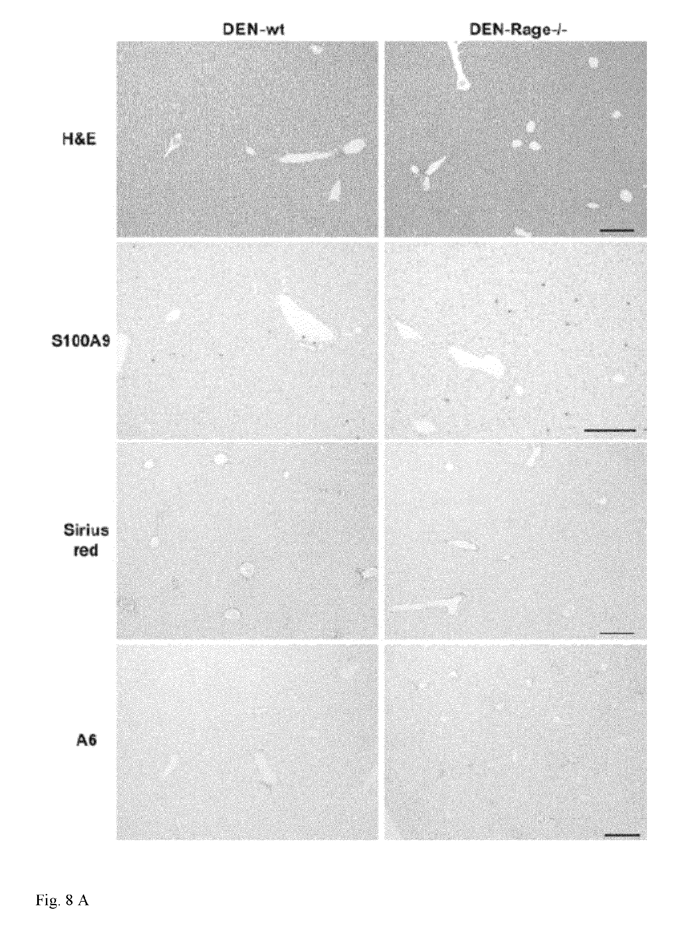

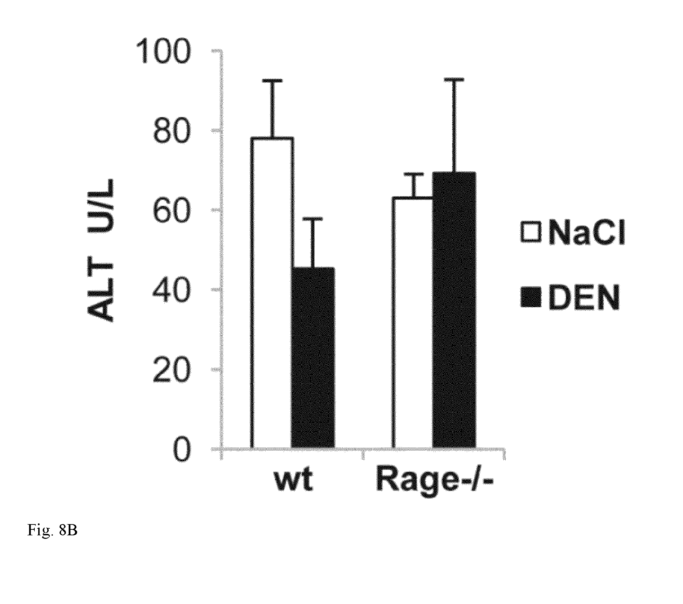

**Figure 8****:** Analysis of inflammation, damage and oval cell activation in pre-malignant DEN-treated wt and *Rage*^{-/-} mice. **A** Immunohistochemical analysis of liver sections from wt and *Rage*^{-/-} mice 6 months upon DEN injection. Hematoxilin and eosin (H&E), S100A9, Sirius red and A6 staining (n=5, respectively; bar, 200µm). **B** ALT serum levels in wt and *Rage*^{-/-}mice 6 months upon DEN injection. Bars represent average ALT serum levels (units/liter) of each mouse group (n=5, respectively); error bars represent SD; *P<0.05.

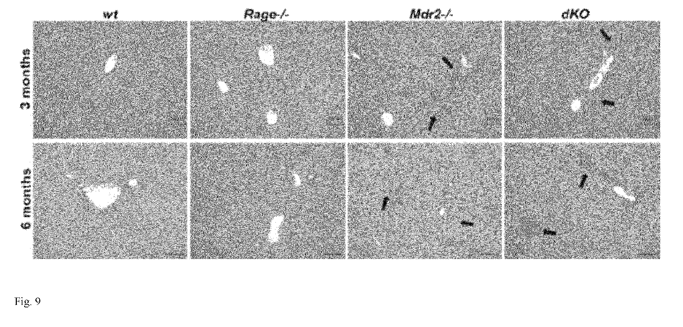

**Figure 9****:** *Mrd2*^{-/-} and *dKO* liver histology. H&E staining of 3 and 6 month old wt, *Rage*^{-/-}*, Mdr2*^{-/-} and *dKO* paraffin liver sections (n=5, respectively). Black arrows: infiltrating inflammatory cells (bar, 200µm).

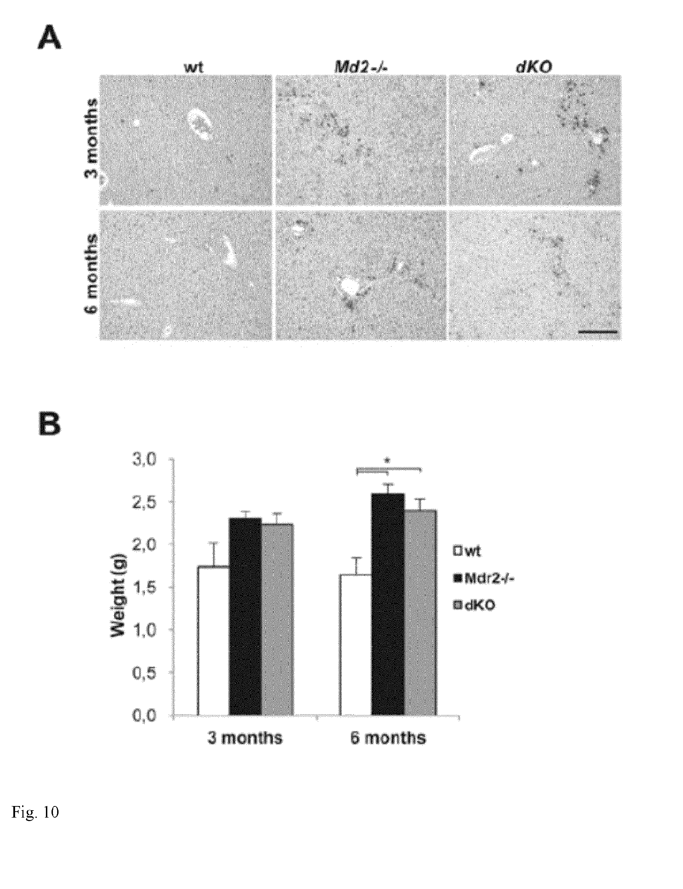

**Figure 10****:** Analysis on *Mdr2*^{-/-} and *dKO* livers. **A** IHC for S 100A8 on paraffin liver sections from 3 and 6 month old wt, *Mdr2*^{-/-} and *dKO* mice (n=5, respectively). Bar, 200µm. **B** Liver weight analysis on 3 and 6 month old wt, *Mdr2*^{-/-} and *dKO* mice (n=5, respectively). Error bars represent SD, * p<0.05.

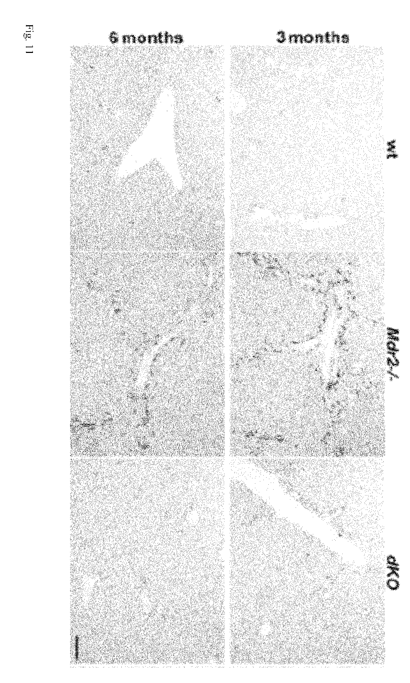

**Figure 11****:** Activation of oval cells. Immunohistochemistry for pan-cytokeratin on liver tissue sections of 3 and 6 month old wt, *Mdr2*^{-/-} and *dKO* mice (n=5, respectively; bar, 200µm).

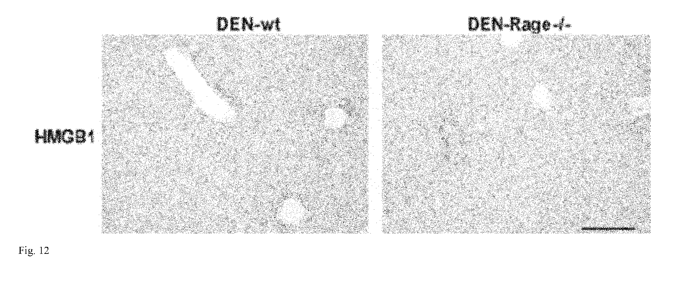

**Figure 12****:** HMGB1 protein levels and cellular location in the liver of wt and *Rage*^{-/-} mice 6 months upon DEN injection. Immunohistochemical analysis of liver sections from wt and *Rage*^{-/-} mice 6 months upon DEN injection, staining for HMGB1 (n=5, respectively; bar, 200µm).

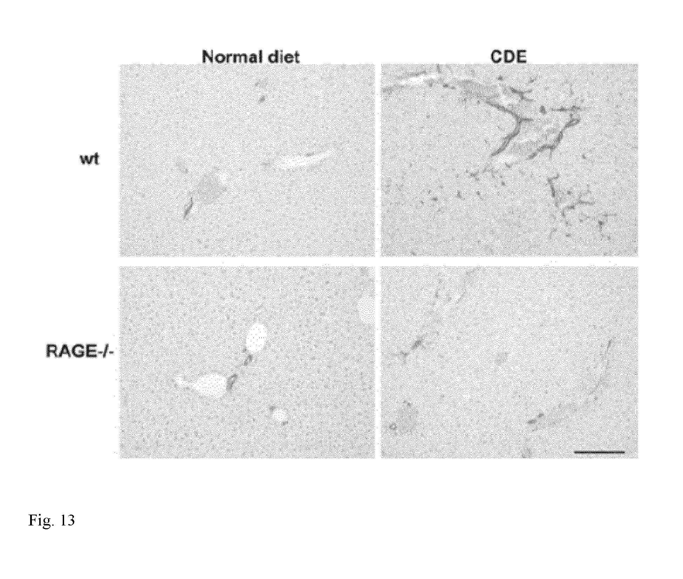

**Figure 13****:** Oval cell activation in *Rage*^{-/-} mice fed a CDE diet. Staining for the A6 antigen on liver sections from wt and *Rage*^{-/-} mice fed a normal or a choline deficient ethionine-supplemented (CDE) diet; bar, 200µm.

### EXAMPLES

The following Examples shall merely illustrate the invention. They shall not be construed, whatsoever, to limit the scope of the invention.

### Example 1: General methods and materials

### Animals and animal work:

Animals were maintained in a specific pathogen-free environment. All animal experiments were performed with aged-matched male mice. The procedures for performing animal experiments were in accordance with the principles and guidelines of the Arbeitsgemeinschaft der Tierschutzbeauftragten in Baden-Württemberg and were approved by the Regierungspräsidium of Karlsruhe, Germany.

*Mdr2*^{-/-} (Pikarsky et al., 2004) and *Rage*^{-/-} (Liliensiek et al., 2004) animals were described previously. For Diethylnitrosamine (DEN) treatment, 15 day-old male C57B1/6 mice were intraperitoneally injected with 10mg/kg DEN and sacrificed 6 and 12 months after injection. For the Choline Deficient Ethionine-supplemented diet (CDE), 5 week-old male wt C57B1/6 mice were fed a choline deficient chow (MP Biomedicals). 0.165% DL-ethionine (Sigma) was provided with the drinking water (Akhurst et al., 2001). After 1 week of treatment mice were randomized, divided in 2 groups and intraperitoneally injected with sRAGE (100µg) or with saline respectively every 2 days for 14 days, thereafter all mice were sacrificed. A four week CDE regime was used for wt and *Rage*^{-/-} mice. ALT activity was measured using an Olympus AU 400 System (measurement rage: 3 - 1000U/l). Isolation of liver cell fractions was performed, following collagenase perfusion, by 20% / 50% discontinuous Percoll gradient followed by MACS separation with CD45 MicroBeads to deplete from the OC fraction the CD45-positive cells.

### Reagents:

Full-length, LPS-free recombinant HMGB1 protein was from *R&D systems*. HMGB1 ELISA was from *Shino-Test Corporation;* Rage and scrambled mouse siRNAs were from *Santa Cruz Biotechnology*. sRAGE was prepared as described (Frommhold et al., 2010).

### Immunohistochemistry and tissue staining:

Tissue fixation, paraffin embedding and subsequent immunohistochemistry staining was performed as previously described (Nemeth et al., 2009) with the following anti-mouse antibodies: phospho-NFκB p65 (1:50, Santa Cruz, sc-101749), S100a8 (1:100; Santa Cruz, sc-8113), S100a9 (1:200; Santa Cruz, sc-8115), Myeloperoxidase (cryo sections 1:100; NeoMarker, RB-373), HMGB1 (1:100; Abcam, ab18256), Pan-CK (1:400; DAKO, Z0622) and A6 (1:100, kind gift of V. Factor). Sirius Red staining was performed as follows: the deparaffinized, rehydrated paraffin sections were stained for 1 hour with picro-sirius red solution (Sigma-Aldrich), washed in 0.5% acetic acid, dehydrated and mounted. At least five mice for each genotype were analyzed and representative pictures are shown.

### Cell culture experiments:

BMOL cells, kindly provided by Prof. George Yeoh, were cultured as described in (Tirnitz-Parker et al., 2007). BMOL cells were transfected with siRNA oligos for RAGE (Ambion) with Hyperfect (Qiagen). Cell counting and cell stimulation was performed as follows: BMOL cells were seeded in 6-well plates (7.5 or 15x10⁴) and grown in complete medium. After 24 hours, cells were starved with serum-free (SF) medium for 16 h and subsequently treated with SF medium, medium with the addition of 2% FBS or SF medium containing 30ng/ml HMGB1. Cells were lysed (for western blot analysis) or counted at the indicated times. Western blotting was performed with the following antibodies: anti-phosphorylated AKT (Cell Signaling, cat. 9271), anti-β-actin (Sigma, clone ac15, cat. # A5441), anti-cyclin D1 (Sigma, clone DCS-6, cat. # C7464), anti-phosphorylated ERK1/2 (Cell Signaling, cat. # 9101), anti-total ERK1/2 (Cell Signaling, cat. # 9102), anti-RAGE (Abcam, cat. ab3611). All experiments were performed at least three times in duplicate.

### RNA preparation and quantitative real-time polymerase chain reaction analysis:

Total RNA extraction was performed according to the manufacturer's instructions using peqGOLD RNAPure (peqlab Biotechnologie). Quantitative real-time polymerase chain reaction (RQ-PCR) analysis was performed as described previously (Gebhardt et al., 2008). Primers used for RQ-PCR analysis were: mouse TNFα: mTNFa_JT_F (5'-GCC TCT TCT CAT TCC TGC TT-3', SEQ ID NO: 4) and mTNFa_JT_R (5'-CTC CTC CAC TTG GTG GTT TG-3', SEQ ID NO: 5), mouse HPRT: mHPRT_for (5'-CTG GTT AAG CAG TAC AGC CCC-3', SEQ ID NO: 6) and mHPRT_rev (5'-CAA AAG TCT GGG GAC GCA GC-3', SEQ ID NO: 7), mouse RAGE: mRage-3/5-F (5'-ACAGGCGAGGGAAGGAGGTC-3', SEQ ID NO: 8) and mRage-3/5-R (5'-TTTGCCATCGGGAATCAGAAG-3', SEQ ID NO: 9), Collagen: Collal for (5'-CACCCTCAAGAGCCTGAGTC-3', SEQ ID NO: 10) and Collal rev (5'-GTTCGGGCTGATGTACCAGT-3', SEQ ID NO: 11). Target gene cycle of threshold values were normalized to the corresponding cycle of threshold values of *Hprt* using the change in cycle of threshold method. The cDNA obtained from at least five different mice for each genotype group was analyzed. Each cDNA was analyzed in triplicates.

### Digital images and statistical analysis:

Digital Images were elaborated using Photoshop CS3 (Adobe); the luminosity of brightest and dimmest pixel in each channel were adjusted to obtain the best visual reproduction, taking care to maintain linearity in the brightness scale. Error bars represent SD except were indicated. Pair wise comparisons between continuous data were done using unpaired two-tailed Student's t test.

### Example 2: Rage is dispensable for Diethylnitrosamine-induced hepatocarcinogenesis

In order to elucidate the role played by Rage in liver tumor formation, wt and *Rage*-deficient (*Rage*^{-/-}) mice were treated with the hepatocarcinogen Diethylnitrosamine (DEN). DEN injection induces DNA-carcinogen adducts, DNA-strand breaks and promotes hepatocyte necrosis and subsequent proliferation, finally resulting in cirrhosis-free HCCs (Chakraborty et al., 2007; Tatematsu et al., 1988). Age and sex-matched wt and *Rage*^{-/-} mice were injected with either DEN (10 mg/kg) or with saline (n=8 each), and 12 months upon injection, mice were sacrificed, livers were harvested and subjected to histological analysis. Both, DEN-treated wt (wtDEN) and *Rage*^{-/-} (*Rage*^{*-*/*-*}DEN) mice developed enlarged livers with multiple dysplastic nodules and malignant tumors, whereas saline treated mice were devoid of lesions (Fig. 7A). Although, the histopathological analysis revealed a slight decrease in the percentage total of HCCs in *Rage*^{*-*/*-*}DEN compared to wtDEN mice (Fig. 7B), no major difference in tumor size (Fig. 7C) or in multiplicity (Fig. 7D) between both genotypes was found.

### Example 3: Hepatocarcinogenesis is impaired in Mdr2^{-/-} Rage^{-/-} double knock-out mice

In contrast to the DEN model, ablation of the *Mdr2*^{-/-} gene promotes hepatocarcinogenesis by the development of chronic liver inflammation and injury. Advantage was taken of this model to investigate the role of Rage in inflammation-driven liver hepatocarcinogenesis and crossed *Rage*^{-/-} mice with the *Mdr2*^{-/-} mouse strain. *Mdr2*^{-/-} *Rage*^{-/-} double knock-out (*dKO*) mice were viable and produced offspring in a Mendelian ratio. At 15 months of age, wt, *Mdr2*^{-/-} and *dKO* mice (n = 10 mice for each group) were sacrificed and livers were analyzed for the presence of neoplastic lesions by histological inspection of H&E stained tissue sections. As expected wt livers had normal size and weight and did not present any focal lesion, while *Mdr2*^{-/-} mice had enlarged livers that developed multiple HCCs and dysplastic nodules (Fig.1A). Pathological grading of tumors from *Mdr2*^{-/-} mice ranged from well differentiated (G1), moderately (G2) up to poorly differentiated (G3), according to the Armed Forces Institute of Pathology grading system. In contrast, *dKO* mice developed mainly dysplastic nodules (Fig. 1 A-B) and only two *dKO* mice exhibited one single HCC classified as moderately differentiated (G2), respectively. Interestingly, while the percentage total of mice, which were free of lesions was comparable between *Mdr2*^{-/-} (28%) and *dKO* (30%) mice, tumor multiplicity and size were significantly higher in *Mdr2*^{-/-} mice. In fact, *Mdr2*^{-/-} mice showed enrichment in HCCs (61%) whereas the major part of lesions (50%) in *dKO* mice was identified as premalignant dysplastic nodules (Fig. 1B). In particular, *dKO* mice showed fewer and smaller liver lesions that did not exceed 12 mm in diameter, whereas lesions in *Mdr2*^{-/-} mice were bigger in size (up to 20 mm in diameter) and in number (Fig.1C). Furthermore, *dKO* mice showed significant less multifocal tumorigenesis compared to *Mdr2*^{-/-}mice (*dKO* 1.25 ± 1.42 (SD) vs. *Mdr2*^{-/-} 4 ± 1.78 (SD); *P*< .*001*; Fig.1D).

### Example 4: Rage ablation does not affect recruitment of inflammatory cells to the liver

As *Rage*^{-/-} mice have already been shown to display impaired inflammation in two mouse models of tumorigenesis (Gebhardt et al., 2008; Turovskaya et al., 2008), it was investigated whether Rage ablation had an impact on liver histology and recruitment of inflammatory cells in the pre-malignant phase upon DEN application or in *Mdr2*^{-/-} mice. In line with previous findings, tissue sections from wtDEN and *Rage*^{*-*/*-*}DEN mice 6 months after DEN injection did not show any sign of altered liver architecture or inflammation, as determined by histological analysis (Fig. 8A). Moreover, immunohistochemical staining for S100A9 (Fig. 8A) and S100A8, two proteins abundantly expressed in inflammatory cells (Gebhardt et al., 2008), as for CD3-positive T cells, displayed an almost absent recruitment of inflammatory cells in both wtDEN and *Rage*^{*-*/}*⁻DEN* liver tissue.

In contrast to DEN treated animals, pre-malignant liver sections from three and six month old *Mdr2*^{*-*/*-*} and *dKO* mice displayed progressive periductular inflammation and a broad rim of periductular extracellular matrix, which was not detected in wt and *Rage*^{*-*/*-*} animals (Fig. 9). Indeed, staining of tissue sections obtained from three and six month-old wt, *Mdr2*^{*-*/*-*} and *dKO* livers for S100A9 (Fig.2A) and S100A8 (Fig. 10A) revealed highly increased levels of S100A8/A9-positive inflammatory cells in liver sections of both *Mdr2*^{*-*/*-*} and *dKO* mice compared to wt controls (Fig. 2A). Yet, no significant difference was found between *Mdr2*^{*-*/-}and *dKO* liver sections. These results were further confirmed by qRT-PCR analysis. Similar data were also obtained by IHC staining of liver sections with an anti-myeloperoxidase (MPO) antibody (Fig. 2B), a specific marker of myeloid cells and for CD3-positive T cells, suggesting that Rage deficiency has no major impact on the recruitment of inflammatory cells to the liver in the *Mdr2*^{*-*/*-*} mouse model.

Accordingly, liver and liver/body weight measurement revealed a slight increase in *Mdr2*^{*-*/-}and *dKO* mice compared to wt at three months of age. This increase became significant after six months (wt 1.65g ± 0.20 (SD); *Mdr2*^{*-*/*-*} 2.60g ± 0.10 (SD) and *dKO* 2.40g ± 0.13 (SD); P< 0.05; Fig. 10B). However, no significant reduction in the liver weight of *dKO* compared to *Mdr2*^{*-*/*-*} mice could be determined. Finally, we measured TNFα expression in livers of three and six month-old wt, *Mdr2*^{*-*/*-*} and *dKO* mice (n=5 for each genotype) by quantitative RT-PCR. Compared to wt controls, *Mdr2*^{*-*/*-*} and *dKO* animals showed a significant increase in TNFα transcript levels at both time points but significant differences in TNFα transcript levels were not observed comparing *Mdr2*^{*-*/*-*} and *dKO* livers (Fig. 2C, P<0.01). These results were further confirmed by ELISA.

### Example 5: Impaired liver damage and oval cell activation in Mdr2^{-/}⁻ Rage^{-/-} double knock-out mice

Rage blockade has already been shown to attenuate injury and toxicity in two models of liver damage (Ekong et al., 2006; Zeng et al., 2004). While 6 month old wtDEN and *Rage*^{-/-}DEN mice displayed no sign of liver fibrosis (Fig. 8A) nor of increased ALT serum levels (Fig. 8B), quantification of serum samples of three and six month-old mice showed significantly higher ALT levels in *Mdr2*^{*-*/*-*} and *dKO* mice compared to wt controls. However, *dKO* mice had reduced ALT levels at three and six months of age compared to *Mdr2*^{*-*/*-*} animals (Fig. 3A). AST analysis revealed comparable results. Chronic liver damage promotes development of fibrosis, a prerequisite for HCC development (Sanyal et al., 2010). Sirius Red staining of *Mdr2*^{*-*/*-*} liver sections displayed periportal and septal fibrosis both at three and six months of age. Interestingly, *dKO* livers displayed only a mild fibrosis at three months that was slightly increased at six months of age (Fig. 3B). Impaired fibrosis in *dKO* livers was further confirmed by decreased collagen1α1 expression detected by quantitative RT-PCR (Fig. 3C).

In cases of chronic and severe liver damage, oval cells, liver progenitor cells, get activated (Alison, 2005). Since it was shown that *dKO* mice display impaired liver damage and fibrosis, the question was addressed whether Rage ablation affects oval cell activation. Liver sections of three and six month-old wt, *Mdr2*^{*-*/*-*} and *dKO* mice were stained with the anti-A6 antibody (Fig. 3D) and with pan-cytokeratin (Fig. 11), two indicative oval cell markers (Engelhardt et al., 1990; Haybaeck et al., 2009; Kofman et al., 2005). As expected, positive staining in wt liver sections was restricted to the portal tracts, whereas intense staining for activated oval cells invading the liver parenchyma was found in three and six month-old *Mdr2*^{*-*/*-*} livers (Fig. 3D and Fig. 11). The presence of activated oval cells was closely associated with inflammatory infiltrates and fibrotic regions. Importantly, oval cell activation was strongly impaired in *dKO* liver sections although inflammatory infiltrates and fibrotic areas were present (Fig. 3D). It is also worth mentioning that IHC analysis for the A6 antigen (Fig. 8A) and pan-cytokeratin (data not shown) on liver sections of 6 month old wtDEN and *Rage*^{*-*/-}DEN mice revealed a restricted staining in both cases to the portal fields, demonstrating no activation of oval cells in the pre-malignant liver in these tumor model. Taken together, these data demonstrate an obvious delay in the onset of liver damage and in activation of oval cells in the pre-malignant phase of *dKO* compared to *Mdr2*^{*-*/*-*} mice.

### Example 6: Rage expression and function on oval cell proliferation in vitro

So far, our results indicate that Rage specifically plays a role in liver carcinogenesis when HCC formation is preceded by chronic inflammation, tissue damage and oval cell activation. In fact, when comparing the activation of oval cells in tumor bearing livers derived from the *Mdr2*^{*-*/*-*} and DEN mouse models, a strong oval cell activation in the parenchyma of *Mdr2*^{*-*/-}mice (Fig. 4A), which was almost absent in DEN-treated mice (Fig. 4B), was detected.

To better understand the role played by Rage in the activation of oval cells, Rage expression was analyzed in hepatocytes, inflammatory (CD45 positive) and oval cells isolated from livers of mice fed with a Choline Deficient Ethionine supplemented diet (CDE), a dietary regime which induces liver injury and strong oval cell activation (Akhurst et al., 2001; Knight et al., 2005). Quantitative RT-PCR and western blot analysis revealed that Rage was significantly expressed in inflammatory cells but barely detectable in hepatocytes. Noteworthy, the highest Rage expression was detected in oval cells (Fig. 4C and D), supporting the assumption that Rage represents a direct regulator of oval cell activation. Furthermore, *in vitro* silencing of Rage expression in BMOL cells (Fig. 4D), an oval cell line (Jellicoe et al., 2008; Tirnitz-Parker et al., 2007), caused a substantial decrease in cell growth when compared to BMOL cells transfected with scrambled oligos (Fig. 4E).

### Example 7: HMGB1 promotes oval cell proliferation

Next, the question was addressed, which ligand of Rage may drive oval cell activation in the *Mdr2*^{*-*/*-*} mouse model. Quantification of protein levels of known ligands such as CML, one of the most abundant AGE structure, and S100A8 and S100A9 in liver and serum samples respectively, revealed comparable amounts in 3 and 6 month-old wt, *Mdr2*^{*-*/*-*} and *dKO* mice. However, HMGB1 serum levels were significantly elevated in *Mdr2*^{*-*/*-*} and *dKO* mice compared to wt controls both at 3 and 6 months of age (Fig. 5A). Accordingly, IHC staining for HMGB1 was exclusively nuclear in hepatocytes of wt controls, whereas liver sections of *Mdr2*^{*-*/*-*} and *dKO* mice displayed strong HMGB1 expression in infiltrating inflammatory cells, accompanied by cytoplasmic relocation of HMGB1 in adjacent hepatocytes (Fig. 5B). These data suggest that activated inflammatory cells promote HMGB1 secretion in hepatocytes and thereby promote liver damage and activation of oval cells. In line with this assumption, HMGB1 staining of liver sections from wt and *Rage*^{*-*/*-*} mice 6 months upon DEN injection displayed an exclusive nuclear staining (Fig. 12).

Next, it was investigated whether HMGB 1 exerts an effect on oval cell activation and treated BMOL cells with recombinant HMGB1 (30 ng/ml). Indeed, we found increased ERK1/2 phosphorylation (Fig. 5C), induced expression of the cell cycle regulator cyclin D1 (Fig. 5D) and accelerated cell proliferation (Fig. 5E) upon HMGB1 application compared to control treated cells. Accordingly, in the presence of the MEK1/2 inhibitor UO126 (10 µM) HMGB 1-induced cyclin D1 expression (Fig. 5D) and BMOL cell growth (Fig. 5F) were attenuated, supporting the hypothesis that ERK1/2-dependent cyclin D1 expression is, at least in part, responsible for the HMGB1-induced activation of BMOL cells.

### Example 8: sRAGE treatment impairs oval cell activation in CDE-treated mice

Finally, it was addressed whether engagement of Rage by its ligands, in particular HMGB 1, plays a direct role in oval cell activation during liver injury *in vivo.* Wt mice were treated for 3 weeks with a CDE diet and after the first week of CDE treatment, mice were injected every second day with either soluble RAGE (sRAGE, 100µg/mouse), a decoy receptor (Frommhold et al., 2010), or saline. After 2 weeks of sRAGE/saline treatment mice were sacrificed and livers were harvested for histological analysis. Quantification of serum ALT levels revealed accelerated liver damage in CDE-treated mice compared to controls that were fed a normal diet. However no significant difference was measured between CDE-treated control (CDE-control) and sRAGE-injected (CDE-sRAGE) mice (Fig. 6A). Intriguingly, CDE-sRAGE liver sections displayed impaired activation of oval cells, as determined by IHC staining for A6 (Fig. 6B) and pan-CK, when compared to CDE-control animals, while no activation of oval cells was detectable in sRAGE- and control-injected mice that were fed a normal diet. These data demonstrated a crucial function of Rage signaling in oval cell activation upon liver tissue damage in vivo, which was further supported by impaired oval cell activation in *Rage*^{*-*/-}compared to wt mice when applying a 4 week CDE diet (Fig. 13).

### The following references were cited throughout the specification

Akhurst, B., Croager, E. J., Farley-Roche, C. A., Ong, J. K., Dumble, M. L., Knight, B., and Yeoh, G. C. (2001). A modified choline-deficient, ethionine-supplemented diet protocol effectively induces oval cells in mouse liver. Hepatology 34, 519-522.
Albayrak, A., Uyanik, M. H., Cerrah, S., Altas, S., Dursun, H., Demir, M., and Uslu, H. (2010). Is HMGB1 a new indirect marker for revealing fibrosis in chronic hepatitis and a new therapeutic target in treatment? Viral immunology 23, 633-638.
Alison, M. R. (2005). Liver stem cells: implications for hepatocarcinogenesis. Stem Cell Rev 1, 253-260.
Andrassy, M., Volz, H. C., Igwe, J. C., Funke, B., Eichberger, S. N., Kaya, Z., Buss, S., Autschbach, F., Pleger, S. T., Lukic, I. K., et al. (2008). High-mobility group box-1 in ischemia-reperfusion injury of the heart. Circulation 117, 3216-3226.
Basta, G., Navarra, T., De Simone, P., Del Turco, S., Gastaldelli, A., and Filipponi, F. (2011). What is the role of the receptor for advanced glycation end products-ligand axis in liver injury? Liver transplantation: official publication of the American Association for the Study of Liver Diseases and the International Liver Transplantation Society 17, 633-640.
Bernstein 2001, Nature 409, 363-366.
Bierhaus A, Haslbeck KM, Humpert PM, Liliensiek B, Dehmer T, Morcos M, Sayed AA, Andrassy M, Schiekofer S, Schneider JG, Schulz JB, Heuss D, Neundorfer B, Dierl S, Huber J, Tritschler H, Schmidt AM, Schwaninger M, Haering HU, Schleicher E, Kasper M, Stem DM, Arnold B, Nawroth PP (2004): Loss of pain perception in diabetes is dependent on a receptor of the immunoglobulin superfamily. J Clin Invest, 114:1741-1751.
Bierhaus, A., and Nawroth, P. P. (2009). Multiple levels of regulation determine the role of the receptor for AGE (RAGE) as common soil in inflammation, immune responses and diabetes mellitus and its complications. Diabetologia 52, 2251-2263.
Block, T. M., Mehta, A. S., Fimmel, C. J., and Jordan, R. (2003). Molecular viral oncology of hepatocellular carcinoma. Oncogene 22, 5093-5107.
Bock 1992, Nature 355 (6360): 564-6.
Boyd, J. H., Kan, B., Roberts, H., Wang, Y., and Walley, K. R. (2008). S100A8 and S100A9 mediate endotoxin-induced cardiomyocyte dysfunction via the receptor for advanced glycation end products. Circ Res 102, 1239-1246.
Bruix J, Boix L, Sala M, Llovet JM (2004): Focus on hepatocellular carcinoma. Cancer Cell, 5:215-219.
Buttari B, Profumo E, Capozzi A, Facchiano F, Saso L, Sorice M, Rigano R (2011): Advanced glycation end products of human beta glycoprotein I modulate the maturation and function of DCs. Blood, 117:6152-6161.
Calogero, S., Grassi, F., Aguzzi, A., Voigtlander, T., Ferrier, P., Ferrari, S., and Bianchi, M. E. (1999). The lack of chromosomal protein Hmg1 does not disrupt cell growth but causes lethal hypoglycaemia in newborn mice. Nat Genet 22, 276-280.
Cataldegirmen, G., Zeng, S., Feirt, N., Ippagunta, N., Dun, H., Qu, W., Lu, Y., Rong, L. L., Hofmann, M. A., Kislinger, T., et al. (2005). RAGE limits regeneration after massive liver injury by coordinated suppression of TNF-alpha and NF-kappaB. J Exp Med 201, 473-484.
Chakraborty, T., Chatterjee, A., Rana, A., Srivastawa, S., Damodaran, S., and Chatterjee, M. (2007). Cell proliferation and hepatocarcinogenesis in rat initiated by diethylnitrosamine and promoted by phenobarbital: potential roles of early DNA damage and liver metallothionein expression. Life sciences 81, 489-499.
de Lima, V. M., Oliveira, C. P., Alves, V. A., Chammas, M. C., Oliveira, E. P., Stefano, J. T., de Mello, E. S., Cerri, G. G., Carrilho, F. J., and Caldwell, S. H. (2008). A rodent model of NASH with cirrhosis, oval cell proliferation and hepatocellular carcinoma. J Hepatol 49, 1055-1061.
Dimond 2010, Genetic Engineering & Biotechnology News 30 (6):1.
Dumitriu IE, Baruah P, Valentinis B, Voll RE, Herrmann M, Nawroth PP, Arnold B, Bianchi ME, Manfredi AA, Rovere-Querini P (2005): Release of high mobility group box 1 by dendritic cells controls T cell activation via the receptor for advanced glycation end products. J Immunol, 174:7506-7515.
Ekong, U., Zeng, S., Dun, H., Feirt, N., Guo, J., Ippagunta, N., Guarrera, J. V., Lu, Y., Weinberg, A., Qu, W., et al. (2006). Blockade of the receptor for advanced glycation end products attenuates acetaminophen-induced hepatotoxicity in mice. J Gastroenterol Hepatol 21, 682-688.
Elbashir 2001, Genes Dev. 15, 188-200.
Elbashir 2001a, Nature 411: 494-498.
Ellington 1990, Nature 346 (6287): 818-22).
El-Serag HB, Rudolph KL (2007): Hepatocellular carcinoma: epidemiology and molecular carcinogenesis. Gastroenterology, 132:2557-2576.
Engelhardt, N. V., Factor, V. M., Yasova, A. K., Poltoranina, V. S., Baranov, V. N., and Lasareva, M. N. (1990). Common antigens of mouse oval and biliary epithelial cells. Expression on newly formed hepatocytes. Differentiation 45, 29-37.
Evankovich, J., Cho, S. W., Zhang, R., Cardinal, J., Dhupar, R., Zhang, L., Klune, J. R., Zlotnicki, J., Billiar, T., and Tsung, A. (2010). High mobility group box 1 release from hepatocytes during ischemia and reperfusion injury is mediated by decreased histone deacetylase activity. J Biol Chem 285, 39888-39897.
Fehrenbach, H., Kasper, M., Tschernig, T., Shearman, M. S., Schuh, D., and Muller, M. (1998). Receptor for advanced glycation endproducts (RAGE) exhibits highly differential cellular and subcellular localisation in rat and human lung. Cell Mol Biol (Noisy-le-grand) 44, 1147-1157.
Fire 1998, Nature 391:806-811.
Fire 1999, Trends Genet. 15, 358-363.
Frommhold, D., Kamphues, A., Hepper, I., Pruenster, M., Lukic, I. K., Socher, I., Zablotskaya, V., Buschmann, K., Lange-Sperandio, B., Schymeinsky, J., et al. (2010). RAGE and ICAM-1 cooperate in mediating leukocyte recruitment during acute inflammation in vivo. Blood 116, 841-849.
Frommhold, D., Kamphues, A., Dannenberg, S., Buschmann, K., Zablotskya, V., Tschada, R., Lange-Sperandio, B., Nawroth, P. P., Poeschl, J., Bierhaus, A., and Sperandio, M. (2011). RAGE and ICAM-1 differentially control leukocyte recruitment during acute inflammation in a stimulus-dependent manner. BMC immunology 12, 56.
Gebhardt, C., Riehl, A., Durchdewald, M., Nemeth, J., Furstenberger, G., Muller-Decker, K., Enk, A., Arnold, B., Bierhaus, A., Nawroth, P. P., et al. (2008). RAGE signaling sustains inflammation and promotes tumor development. J Exp Med 205, 275-285.
Ghavami, S., Kerkhoff, C., Chazin, W. J., Kadkhoda, K., Xiao, W., Zuse, A., Hashemi, M., Eshraghi, M., Schulze-Osthoff, K., Klonisch, T., and Los, M. (2008). S100A8/9 induces cell death via a novel, RAGE-independent pathway that involves selective release of Smac/DIABLO and Omi/HtrA2. Biochim Biophys Acta 1783, 297-311.
Ghavami S, Rashedi I, Dattilo BM, Eshraghi M, Chazin WJ, Hashemi M, Wesselborg S, Kerkhoff C, Los M (2008a): S100A8/A9 at low concentration promotes tumor cell growth via RAGE ligation and MAP kinase-dependent pathway. J Leukoc Biol 2008, 83:1484-1492.
Grivennikov, S. I., Greten, F. R., and Karin, M. (2010). Immunity, inflammation, and cancer. Cell 140, 883-899.
Guo, H. F., Liu, S. X., Zhang, Y. J., Liu, Q. J., Hao, J., and Gao, L. X. (2011). High mobility group box 1 induces synoviocyte proliferation in rheumatoid arthritis by activating the signal transducer and activator transcription signal pathway. Clin Exp Med 11, 65-74.
Hamilton 1999, Science 286, 950-952.
Hammond 2000, Nature 404, 293-296.
Hammond 2001, Nature Rev. Genet. 2, 1110-1119.
Haybaeck, J., Zeller, N., Wolf, M. J., Weber, A., Wagner, U., Kurrer, M. O., Bremer, J., Iezzi, G., Graf, R., Clavien, P. A., et al. (2009). A lymphotoxin-driven pathway to hepatocellular carcinoma. Cancer Cell 16, 295-308.
Hean J, Weinberg MS (2008). "The Hammerhead Ribozyme Revisited: New Biological Insights for the Development of Therapeutic Agents and for Reverse Genomics Applications". In Morris KL. RNA and the Regulation of Gene Expression: A Hidden Layer of Complexity. Norfolk, England: Caister Academic Press.
Hiwatashi, K., Ueno, S., Abeyama, K., Kubo, F., Sakoda, M., Maruyama, I., Hamanoue, M., Natsugoe, S., and Aikou, T. (2008). A novel function of the receptor for advanced glycation end-products (RAGE) in association with tumorigenesis and tumor differentiation of HCC. Annals of surgical oncology 15, 923-933.
Hoppe-Seyler 2000, J Mol Med. 78 (8): 426-30.
Hyogo, H., and Yamagishi, S. (2008). Advanced glycation end products (AGEs) and their involvement in liver disease. Curr Pharm Des 14, 969-972.
Jellicoe, M. M., Nichols, S. J., Callus, B. A., Baker, M. V., Barnard, P. J., Berners-Price, S. J., Whelan, J., Yeoh, G. C., and Filipovska, A. (2008). Bioenergetic differences selectively sensitize tumorigenic liver progenitor cells to a new gold(I) compound. Carcinogenesis 29, 1124-1133.
Jiang, W., Wang, Z., Li, X., Fan, X., and Duan, Y. (2011). High-mobility Group Box 1 is Associated with Clinicopathologic Features in Patients with Hepatocellular Carcinoma. Pathology oncology research : POR.
Knight, B., Matthews, V. B., Akhurst, B., Croager, E. J., Klinken, E., Abraham, L. J., Olynyk, J. K., and Yeoh, G. (2005). Liver inflammation and cytokine production, but not acute phase protein synthesis, accompany the adult liver progenitor (oval) cell response to chronic liver injury. Immunology and cell biology 83, 364-374.
Knight, B., Yeoh, G. C., Husk, K. L., Ly, T., Abraham, L. J., Yu, C., Rhim, J. A., and Fausto, N. (2000). Impaired preneoplastic changes and liver tumor formation in tumor necrosis factor receptor type 1 knockout mice. J Exp Med 192, 1809-1818.
Kofman, A. V., Morgan, G., Kirschenbaum, A., Osbeck, J., Hussain, M., Swenson, S., and Theise, N. D. (2005). Dose- and time-dependent oval cell reaction in acetaminophen-induced murine liver injury. Hepatology 41, 1252-1261.
Kostova, N., Zlateva, S., Ugrinova, I., and Pasheva, E. (2010). The expression of HMGB1 protein and its receptor RAGE in human malignant tumors. Molecular and cellular biochemistry 337, 251-258.
Liliensiek, B., Weigand, M. A., Bierhaus, A., Nicklas, W., Kasper, M., Hofer, S., Plachky, J., Grone, H. J., Kurschus, F. C., Schmidt, A. M., et al. (2004). Receptor for advanced glycation end products (RAGE) regulates sepsis but not the adaptive immune response. J Clin Invest 113, 1641-1650.
Logsdon, C. D., Fuentes, M. K., Huang, E. H., and Arumugam, T. (2007). RAGE and RAGE ligands in cancer. Curr Mol Med 7, 777-789.
Mauad, T. H., van Nieuwkerk, C. M., Dingemans, K. P., Smit, J. J., Schinkel, A. H., Notenboom, R. G., van den Bergh Weerman, M. A., Verkruisen, R. P., Groen, A. K., Oude Elferink, R. P., and et al. (1994). Mice with homozygous disruption of the mdr2 P-glycoprotein gene. A novel animal model for studies of nonsuppurative inflammatory cholangitis and hepatocarcinogenesis. Am J Pathol 145, 1237-1245.
Needleman and Wunsch J. Mol. Biol. 48:443 (1970 ).
Neeper M, Schmidt AM, Brett J, Yan SD, Wang F, Pan YC, Elliston K, Stern D, Shaw A (1992): Cloning and expression of a cell surface receptor for advanced glycosylation end products of proteins. J Biol Chem, 267:14998-15004.
Nemeth, J., Stein, I., Haag, D., Riehl, A., Longerich, T., Horwitz, E., Breuhahn, K., Gebhardt, C., Schirmacher, P., Hahn, M., et al. (2009). S100A8 and S100A9 are novel nuclear factor kappa B target genes during malignant progression of murine and human liver carcinogenesis. Hepatology 50, 1251-1262.
Palumbo 2004, J Cell Biol 164: 441-449.
Pearson and Lipman Proc. Natl. Acad Sci. (USA) 85: 2444 (1988 ).
Pikarsky, E., Porat, R. M., Stein, I., Abramovitch, R., Amit, S., Kasem, S., Gutkovich-Pyest, E., Urieli-Shoval, S., Galun, E., and Ben-Neriah, Y. (2004). NF-kappaB functions as a tumour promoter in inflammation-associated cancer. Nature 431, 461-466.
Pradere, J. P., Troeger, J. S., Dapito, D. H., Mencin, A. A., and Schwabe, R. F. (2010). Toll-like receptor 4 and hepatic fibrogenesis. Semin Liver Dis 30, 232-244.
Ranzato, E., Patrone, M., Pedrazzi, M., and Burlando, B. (2010). Hmgb1 promotes wound healing of 3 T3 mouse fibroblasts via RAGE-dependent ERK1/2 activation. Cell Biochem Biophys 57, 9-17.
Riehl, A., Nemeth, J., Angel, P., and Hess, J. (2009). The receptor RAGE: Bridging inflammation and cancer. Cell Commun Signal 7, 12.
Saha A, Lee YC, Zhang Z, Chandra G, Su SB, Mukherjee AB (2010): Lack of an endogenous anti-inflammatory protein in mice enhances colonization of B16F10 melanoma cells in the lungs. J Biol Chem, 285:10822-10831.
Sanyal, A. J., Yoon, S. K., and Lencioni, R. (2010). The etiology of hepatocellular carcinoma and consequences for treatment. Oncologist 15 Suppl 4, 14-22.
Sell, S., and Leffert, H. L. (2008). Liver cancer stem cells. J Clin Oncol 26, 2800-2805.
Severi T, van Malenstein H, Verslype C, van Pelt JF (2010): Tumor initiation and progression in hepatocellular carcinoma: risk factors, classification, and therapeutic targets. Acta Pharmacol Sin, 31:1409-1420.
Sharp 2001, Genes Dev. 15,485-490.
Sims, G. P., Rowe, D. C., Rietdijk, S. T., Herbst, R., and Coyle, A. J. (2010). HMGB1 and RAGE in inflammation and cancer. Annu Rev Immunol 28, 367-388.
Sitia G, lannacone M, Muller S, Bianchi ME, Guidotti LG (2007): Treatment with HMGB1 inhibitors diminishes CTL-induced liver disease in HBV transgenic mice. J Leukoc Biol, 81:100-107.
Smith and Waterman Add. APL. Math. 2:482 (1981).
Summerton 1997, Antisense & Nucleic Acid Drug Development 7* (3): 187-95.
Summerton 1999, Biochimica et Biophysica Acta 1489 (1): 141-58.
Taguchi, A., Blood, D. C., del Toro, G., Canet, A., Lee, D. C., Qu, W., Tanji, N., Lu, Y., Lalla, E., Fu, C., et al. (2000). Blockade of RAGE-amphoterin signalling suppresses tumour growth and metastases. Nature 405, 354-360.
Tatematsu, M., Aoki, T., Kagawa, M., Mera, Y., and Ito, N. (1988). Reciprocal relationship between development of glutathione S-transferase positive liver foci and proliferation of surrounding hepatocytes in rats. Carcinogenesis 9, 221-225.
Tirnitz-Parker, J. E., Tonkin, J. N., Knight, B., Olynyk, J. K., and Yeoh, G. C. (2007). Isolation, culture and immortalisation of hepatic oval cells from adult mice fed a choline-deficient, ethionine-supplemented diet. Int J Biochem Cell Biol 39, 2226-2239.
Tsung, A., Sahai, R., Tanaka, H., Nakao, A., Fink, M. P., Lotze, M. T., Yang, H., Li, J., Tracey, K. J., Geller, D. A., and Billiar, T. R. (2005). The nuclear factor HMGB1 mediates hepatic injury after murine liver ischemia-reperfusion. J Exp Med 201, 1135-1143.
Turovskaya, O., Foell, D., Sinha, P., Vogl, T., Newlin, R., Nayak, J., Nguyen, M., Olsson, A., Nawroth, P. P., Bierhaus, A., et al. (2008). RAGE, carboxylated glycans and S100A8/A9 play essential roles in colitis-associated carcinogenesis. Carcinogenesis 29, 2035-2043.
Tuschl 2001, Chem. Biochem. 2, 239-245.
Vainer, G. W., Pikarsky, E., and Ben-Neriah, Y. (2008). Contradictory functions of NF-kappaB in liver physiology and cancer. Cancer letters 267, 182-188.
Vasquez 2002, Quart Rev Biophys 35: 89-107.
Weiss, B. (ed.): Antisense Oligodeoxynucleotides and Antisense RNA : Novel Pharmacological and Therapeutic Agents, CRC Press, Boca Raton, FL, 1997.
Xia, J. R., Liu, N. F., and Zhu, N. X. (2008). Specific siRNA targeting the receptor for advanced glycation end products inhibits experimental hepatic fibrosis in rats. International journal of molecular sciences 9, 638-661.
Yang H, Ochani M, Li J, Qiang X, Tanovic M, Harris HE, Susarla SM, Ulloa L, Wang H, DiRaimo R, Czura CJ, Roth J, Warren HS, Fink MP, Fenton MJ, Andersson U, Tracey KJ (2004): Reversing established sepsis with antagonists of endogenous high-mobility group box 1. Proc Natl Acad Sci U S A, 101:296-301.
Zamore 2000, Cell 101, 25-33.
Zeng, S., Feirt, N., Goldstein, M., Guarrera, J., Ippagunta, N., Ekong, U., Dun, H., Lu, Y., Qu, W., Schmidt, A. M., and Emond, J. C. (2004). Blockade of receptor for advanced glycation end product (RAGE) attenuates ischemia and reperfusion injury to the liver in mice. Hepatology 39, 422-432.
Zeng, S., Dun, H., Ippagunta, N., Rosario, R., Zhang, Q. Y., Lefkowitch, J., Yan, S. F., Schmidt, A. M., and Emond, J. C. (2009). Receptor for advanced glycation end product (RAGE)-dependent modulation of early growth response-1 in hepatic ischemia/reperfusion injury. J Hepatol 50, 929-936.
Zhou, R. R., Zhao, S. S., Zou, M. X., Zhang, P., Zhang, B. X., Dai, X. H., Li, N., Liu, H. B., Wang, H., and Fan, X. G. (2011). HMGB1 cytoplasmic translocation in patients with acute liver failure. BMC Gastroenterol 11, 21.

## Claims

1. An inhibitor of the Receptor for Advanced Glycation-End products (RAGE) for use in treating and/or preventing inflammation- and/or damage-induced cancer.

2. The inhibitor of claim 1, wherein the inhibitor is an antibody which specifically binds to RAGE and inhibits RAGE activity.

3. The inhibitor of claim 2, wherein said antibody when bound to RAGE prevents the binding of a RAGE ligand.

4. The inhibitor of claim 1, wherein said inhibitor is selected from the group consisting of an aptamer which specifically binds to RAGE and inhibit RAGE activity, a soluble RAGE polypeptide, a dominant-negative high mobility group box 1 protein (HMGB 1), and a small molecule antagonist.

5. The inhibitor of claim 1, wherein said inhibitor is an inhibitory nucleic acid.

6. The inhibitor of claim 5, wherein said inhibitory nucleic acid is selected from the group consisting of: an antisense RNA, a ribozyme, a morpholino, a triple-helix forming agent, a siRNA, and a micro RNA.

7. The inhibitor of any one of claims 1 to 6, wherein said inflammation- and/or damage-induced cancer is hepatocarcinoma or hepatocellular carcinoma, cholangiocarcinoma, pancreas cancer, gastrointestinal and colon cancer, esophageal carcinoma.

8. A medicament comprising an inhibitor of RAGE as specified in any one of claims 1 to 7 and a pharmaceutically acceptable carrier.

9. A method for identifying a subject in need of treatment with an inhibitor of RAGE, comprising
a) determining in a sample from said subject the amount of RAGE and / or of at least one marker for inflammation,
b) comparing said amount of RAGE and / or of a marker for inflammation determined in step a) with a reference amount, and
c) identifying a subject in need of treatment with an inhibitor of RAGE.

10. The method of claim 9, wherein said at least one marker for inflammation is a marker for hepatitis.

11. The method of claim 9 or 10, wherein said at least one marker is a RAGE ligand or a serum marker for inflammation.

12. The method of any one of claims 9 to 11, wherein the sample is a liver biopsy sample or a serum sample.

13. The method of any one of claims 9 to 12, wherein said inhibitor of RAGE is an inhibitor of RAGE as defined in any one of claims 1 to 7.

14. A device for identifying a subject in need of treatment with an inhibitor of RAGE, comprising an analyzing unit comprising a) a detection agent for determining the amount of RAGE in a sample from said subject and b) an evaluation unit comprising a data processor having tangibly embedded an algorithm for comparing the amount determined by said analyzing unit to a reference amount for said amount of RAGE and which is capable of generating an output file containing a diagnosis established based on said comparison.

15. A kit adapted to carry out the method of any one of claims 9 to 13, said kit comprising instructions for carrying out the said method, a detection agent capable of determining the amount of RAGE in a sample and a reference which can be used for comparing the determined amount of RAGE from the sample and allowing identifying a subject in need of treatment with an inhibitor of RAGE.


SEQUENCE LISTING

<110>  Deutsches Krebsforschungszentrum
       Ruprecht-Karls-Universität Heidelberg
       The Hebrew University of Jerusalem
       Hadassah Medical Organization

<120>  Inhibitors of Receptor for Advanced Glycation-End products (RAGE)
       for use in treating and/or preventing inflammation- and/or
       damage-induced cancer

<130>  DK10784EP

<160>  3

<170>  PatentIn version 3.5

<210>  1
<211>  1556
<212>  DNA
<213>  Homo sapiens

<400>  1
ctgaaagatg ggggctggag agagggtgca ggccccacct agggcggagg ccacagcagg      60

gagaggggca gacagagcca ggaccctgga aggaagcagg atggcagccg gaacagcagt     120

tggagcctgg gtgctggtcc tcagtctgtg gggggcagta gtaggtgctc aaaacatcac     180

agcccggatt ggcgagccac tggtgctgaa gtgtaagggg gcccccaaga aaccacccca     240

gcggctggaa tggaaactga acacaggccg gacagaagct tggaaggtcc tgtctcccca     300

gggaggaggc ccctgggaca gtgtggctcg tgtccttccc aacggctccc tcttccttcc     360

ggctgtcggg atccaggatg aggggatttt ccggtgccag gcaatgaaca ggaatggaaa     420

ggagaccaag tccaactacc gagtccgtgt ctaccagatt cctgggaagc cagaaattgt     480

agattctgcc tctgaactca cggctggtgt tcccaataag gtagtggaag aaagcaggag     540

aagtagaaaa cggccctgtg aacaggaggt ggggacatgt gtgtcagagg gaagctaccc     600

tgcagggact cttagctggc acttggatgg gaagcccctg gtgcctaatg agaagggagt     660

atctgtgaag gaacagacca ggagacaccc tgagacaggg ctcttcacac tgcagtcgga     720

gctaatggtg accccagccc ggggaggaga tccccgtccc accttctcct gtagcttcag     780

cccaggcctt ccccgacacc gggccttgcg cacagccccc atccagcccc gtgtctggga     840

gcctgtgcct ctggaggagg tccaattggt ggtggagcca gaaggtggag cagtagctcc     900

tggtggaacc gtaaccctga cctgtgaagt ccctgcccag ccctctcctc aaatccactg     960

gatgaaggat ggtgtgccct tgccccttcc ccccagccct gtgctgatcc tccctgagat    1020

agggcctcag gaccagggaa cctacagctg tgtggccacc cattccagcc acgggcccca    1080

ggaaagccgt gctgtcagca tcagcatcat cgaaccaggc gaggaggggc caactgcagg    1140

ctctgtggga ggatcagggc tgggaactct agccctggcc ctggggatcc tgggaggcct    1200

ggggacagcc gccctgctca ttggggtcat cttgtggcaa aggcggcaac gccgaggaga    1260

ggagaggaag gccccagaaa accaggagga agaggaggag cgtgcagaac tgaatcagtc 1320

ggaggaacct gaggcaggcg agagtagtac tggagggcct tgaggggccc acagacagat 1380

cccatccatc agctcccttt tctttttccc ttgaactgtt ctggcctcag accaactctc 1440

tcctgtataa tctctctcct gtataacccc accttgccaa gctttcttct acaaccagag 1500

ccccccacaa tgatgattaa acacctgaca catcttgcaa aaaaaaaaaa aaaaaa 1556

<210> 2
<211> 420
<212> PRT
<213> Homo sapiens

<400> 2

Met Ala Ala Gly Thr Ala Val Gly Ala Trp Val Leu Val Leu Ser Leu
1               5                   10                  15

Trp Gly Ala Val Val Gly Ala Gln Asn Ile Thr Ala Arg Ile Gly Glu
            20                  25                  30

Pro Leu Val Leu Lys Cys Lys Gly Ala Pro Lys Lys Pro Pro Gln Arg
        35                  40                  45

Leu Glu Trp Lys Leu Asn Thr Gly Arg Thr Glu Ala Trp Lys Val Leu
    50                  55                  60

Ser Pro Gln Gly Gly Gly Pro Trp Asp Ser Val Ala Arg Val Leu Pro
65                  70                  75                  80

Asn Gly Ser Leu Phe Leu Pro Ala Val Gly Ile Gln Asp Glu Gly Ile
                85                  90                  95

Phe Arg Cys Gln Ala Met Asn Arg Asn Gly Lys Glu Thr Lys Ser Asn
            100                 105                 110

Tyr Arg Val Arg Val Tyr Gln Ile Pro Gly Lys Pro Glu Ile Val Asp
        115                 120                 125

Ser Ala Ser Glu Leu Thr Ala Gly Val Pro Asn Lys Val Val Glu Glu
    130                 135                 140

Ser Arg Arg Ser Arg Lys Arg Pro Cys Glu Gln Glu Val Gly Thr Cys
145                 150                 155                 160

Val Ser Glu Gly Ser Tyr Pro Ala Gly Thr Leu Ser Trp His Leu Asp
                165                 170                 175

Gly Lys Pro Leu Val Pro Asn Glu Lys Gly Val Ser Val Lys Glu Gln
            180                 185                 190

Thr Arg Arg His Pro Glu Thr Gly Leu Phe Thr Leu Gln Ser Glu Leu
        195                 200                 205

Met Val Thr Pro Ala Arg Gly Gly Asp Pro Arg Pro Thr Phe Ser Cys
    210                 215                 220

Ser Phe Ser Pro Gly Leu Pro Arg His Arg Ala Leu Arg Thr Ala Pro
225                 230                 235                 240

Ile Gln Pro Arg Val Trp Glu Pro Val Pro Leu Glu Glu Val Gln Leu
                245                 250                 255

Val Val Glu Pro Glu Gly Gly Ala Val Ala Pro Gly Gly Thr Val Thr
            260                 265                 270

Leu Thr Cys Glu Val Pro Ala Gln Pro Ser Pro Gln Ile His Trp Met
        275                 280                 285

Lys Asp Gly Val Pro Leu Pro Leu Pro Pro Ser Pro Val Leu Ile Leu
    290                 295                 300

Pro Glu Ile Gly Pro Gln Asp Gln Gly Thr Tyr Ser Cys Val Ala Thr
305                 310                 315                 320

His Ser Ser His Gly Pro Gln Glu Ser Arg Ala Val Ser Ile Ser Ile
                325                 330                 335

Ile Glu Pro Gly Glu Glu Gly Pro Thr Ala Gly Ser Val Gly Gly Ser
            340                 345                 350

Gly Leu Gly Thr Leu Ala Leu Ala Leu Gly Ile Leu Gly Gly Leu Gly
        355                 360                 365

Thr Ala Ala Leu Leu Ile Gly Val Ile Leu Trp Gln Arg Arg Gln Arg
    370                 375                 380

Arg Gly Glu Glu Arg Lys Ala Pro Glu Asn Gln Glu Glu Glu Glu Glu
385                 390                 395                 400

Arg Ala Glu Leu Asn Gln Ser Glu Glu Pro Glu Ala Gly Glu Ser Ser
                405                 410                 415

Thr Gly Gly Pro
            420

<210>  3
<211>  3355
<212>  DNA

<213>  Homo sapiens

<400>  3
ctgaaagatg ggggctggag agagggtgca ggccccacct agggcggagg ccacagcagg      60

gagaggggca gacagagcca ggaccctgga aggaagcagg atggctgccg gaacagcagt     120

tggagcctgg gtgctggtcc tcagtctgtg gggtgagcca ctccctcaac cccactgacc     180

ctccctgcag aaagcacttt aaccccacac cccagtcgtc ctagaacttt tcccagaacc     240

cgaggaagtg cctttcaagg tccctcaccc accctgtcca aattttgtta gccctcattc     300

ccttcctacc cctctaccat ggtgctatct cccaggggca gtagtaggtg ctcaaaacat     360

cacagcccgg attggcgagc cactggtgct gaagtgtaag gggcccccca agaaaccacc     420

ccagcggctg gaatggaaac tggtaagcgg ggctcctgtt gcagcctccc aacttccagg     480

gagaccagca atgatttgga tccccgtcac tctgcctcac agtcctttcc caaaggcctt     540

gcactgttta ggccctgctt ctctgcttct agaacacagg ccggacagaa gcttggaagg     600

tcctgtctcc ccagggagga ggcccctggg acagtgtggc tcgtgtcctt cccaacggct     660

ccctcttcct tccggctgtc gggatccagg atgaggggat tttccggtgc caggcaatga     720

acaggaatgg aaaggagacc aagtccaact accgagtccg tgtctaccgt aagaattcca     780

gggtcttctc caaggcctcc ctcttaccta agaaaaagcc ttcaacccca gccttggccc     840

atgagggcct ctgacttcca ctggcctcat ttccacacac agagtttgag aaccttcaca     900

attacagcct ctgactggat ttttcctcct tcagagattc ctgggaagcc agaaattgta     960

gattctgcct ctgaactcac ggctggtgtt cccaataagg tagtggaaga aagcaggaga    1020

agtagaaaac ggccctgtga acaggaggcg agtgtgtgtg ggtgtgggtg tgtggcatct    1080

ctcattttca aaggattctg aggtcaccac tctttcccca ggtggggaca tgtgtgtcag    1140

agggaagcta ccctgcaggg actcttagct ggcacttgga tgggaagccc ctggtgccta    1200

atgagaaggg tgagtcctaa ggtgcccccc aagctgcctt ctccctgatc tcactcccac    1260

acccaccctg ggataatttg tcttatcctc ccatcatagg agtatctgtg aaggaacaga    1320

ccaggagaca ccctgagaca gggctcttca cactgcagtc ggagctaatg gtgaccccag    1380

cccggggagg agatccccgt cccaccttct cctgtagctt cagcccaggc cttccccgac    1440

accgggcctt gcgcacagcc cccatccagc cccgtgtctg gggtgagcat aggtggggag    1500

ggccccaagc tcacgtgagc acgttctgga agtctgaccc ttagggaaag agggagtcaa    1560

gcccatggcc actgggatca ctcacaagtg taactctcca cctcaaaacc cttccaactc    1620

ccagagcctg tgcctctgga ggaggtccaa ttggtggtgg agccagaagg tggagcagta    1680

gctcctggtg gaaccgtaac cctgacctgt gaagtccctg cccagccctc tcctcaaatc    1740

cactggatga aggatgtgag tgacctggag agaggggctg ggaggtaggg tgaaccataa    1800

ctagcaacag ggagggcaga gggctaacga gggaaaggca ggctaggagc tgaggaggaa    1860

gagagggtat ctgaagatat ggagacaaaa agacaagggt tttgaaatag tctcctctcc 1920

ccttccccca ccagggtgtg cccttgcccc ttccccccag ccctgtgctg atcctccctg 1980

agatagggcc tcaggaccag ggaacctaca gctgtgtggc cacccattcc agccacgggc 2040

cccaggaaag ccgtgctgtc agcatcagca tcatcggtga gacctctccc caagccctac 2100

agaccctggg actagggtgc aggacagcac aggctctaat ttcctgcccc attctggcct 2160

tatccctaac agccacccca cctctccctc catgcaccca cacccaagcc tcccctgccc 2220

cacccaaatt ctgccaagag agcagccaag cctctccctt cttccctctg agctaaaaaa 2280

aggaacagac ggctgggcgc ggtggctcac gcctgtaatc ccaacacttt gggaggctga 2340

ggcgggcaga tcacctgagg tagggagttc gagaccagcc tgaccaacat ggagaaaccc 2400

catttctact aaaaatacaa aattagccag gcatggtggc acatgcctgt aatcccagct 2460

acctgggagg ccagctactt gagaggctga ggcaggagaa ttgcttgaac ccaggaggca 2520

tagattgcga tgagccaaga tcgcaccatt gcatgccagc ctgggcaaca aaagtgaaac 2580

tccatctcaa aaaaaaaaag aaagggaaag actccactgg ggctcccact aaataaccct 2640

ctctcaaccc gaagtcttcc tttctgactg gatccaactt tgtcttccag aaccaggcga 2700

ggaggggcca actgcaggtg aggggtttga taaagtcagg gaagcagaag atagccccca 2760

acacatgtga ctggggggat ggtcaacaag aaaggaatgg tgagtggtgg tggctgtgct 2820

ctcaattttc cctgtctccg tacaggctct gtgggaggat cagggctggg aactctagcc 2880

ctggccctgg ggatcctggg aggcctgggg acagccgccc tgctcattgg ggtcatcttg 2940

tggcaaaggc ggcaacgccg aggagaggag aggtgagtgg agaaagccag acccctcaga 3000

cctagggctt ccaggcagca agcgaagagg ggtcgggggg tggaacgaca acgtgccgca 3060

ttccccccaa tctttctcct caggaaggcc ccagaaaacc aggaggaaga ggaggagcgt 3120

gcagaactga atcagtcgga ggaacctgag gcaggcgaga gtagtactgg agggccttga 3180

ggggcccaca gacagatccc atccatcagc tcccttttct ttttcccttg aactgttctg 3240

gcctcagacc aactctctcc tgtataatct ctctcctgta taaccccacc ttgccaagct 3300

ttcttctaca accagagccc cccacaatga tgattaaaca cctgacacat cttgc 3355